(19) Europäisches Patentamt
European Patent Office
Office européen des brevets

(11) **EP 4 079 719 A1**

(12) **EUROPEAN PATENT APPLICATION**
published in accordance with Art. 153(4) EPC

(43) Date of publication:
**26.10.2022 Bulletin 2022/43**

(21) Application number: **20903121.0**

(22) Date of filing: **18.12.2020**

(51) International Patent Classification (IPC):
$C07C\ 29/34$ (2006.01)     $B01J\ 23/75$ (2006.01)
$B01J\ 23/755$ (2006.01)     $B01J\ 23/80$ (2006.01)
$B01J\ 23/83$ (2006.01)     $B01J\ 23/847$ (2006.01)
$B01J\ 23/883$ (2006.01)     $B01J\ 23/888$ (2006.01)
$B01J\ 23/889$ (2006.01)     $B01J\ 23/89$ (2006.01)
$C07C\ 31/02$ (2006.01)     $C07C\ 31/125$ (2006.01)
$C07B\ 61/00$ (2006.01)

(52) Cooperative Patent Classification (CPC):
**B01J 23/75; B01J 23/755; B01J 23/78;
B01J 23/80; B01J 23/83; B01J 23/84;
B01J 23/847; B01J 23/883; B01J 23/885;
B01J 23/888; B01J 23/889; B01J 23/89;
C07C 29/34; C07C 31/02; C07C 31/125;**     (Cont.)

(86) International application number:
**PCT/JP2020/047424**

(87) International publication number:
**WO 2021/125322 (24.06.2021 Gazette 2021/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.12.2019 JP 2019228249**

(71) Applicant: **Kao Corporation
Chuo-ku
Tokyo 103-8210 (JP)**

(72) Inventors:
• **TOTOKI, Takenori
Wakayama-shi, Wakayama 640-8580 (JP)**
• **TAKADA, Shingo
Wakayama-shi, Wakayama 640-8580 (JP)**

(74) Representative: **Hoffmann Eitle
Patent- und Rechtsanwälte PartmbB
Arabellastraße 30
81925 München (DE)**

(54) **METHOD FOR PRODUCING GUERBET ALCOHOL**

(57)     A method for producing a Guerbet alcohol, including reacting a raw material alcohol having 8 or more and 22 or less carbon atoms, in the presence of a catalyst (A) containing a first component, a second component, and a third component below: first component: copper, second component: one kind selected from the group consisting of cobalt, nickel, molybdenum, and rhenium, and third component: at least one kind selected from the group consisting of elements that are elements belonging to Groups 3 to 10 and 12 of the fourth period of the periodic table and elements belonging to Groups 3 to 7, 11, and 12 of the fifth and sixth periods of the periodic table, and are different from the element selected as the second component.

**(Cont. next page)**

(52) Cooperative Patent Classification (CPC): (Cont.)
**C07C 33/025;** C07B 61/00

**Description**

Field of the Invention

The present invention relates to a method for producing a Guerbet alcohol.

Background of the Invention

It has been widely known that an aliphatic alcohol is reacted in the presence of a base catalyst or in the presence of a base catalyst and a cocatalyst to produce one molecule of a branched dimerized alcohol (i.e., a Guerbet alcohol) through removal of one molecule of water from two molecules of the alcohol, and the reaction has been referred to as Guerbet reaction.

In the case where a primary alcohol is used as the raw material alcohol as an example, it has been estimated that the reaction mechanism of the Guerbet reaction is constituted by the following elementary reactions (1) to (4):

(1) formation of an aldehyde through dehydrogenation of the alcohol,
(2) formation of an $\alpha,\beta$-unsaturated aldehyde through aldol condensation of the aldehyde,
(3) formation of an allyl alcohol through reduction of the $\alpha,\beta$-unsaturated aldehyde, and
(4) formation of a Guerbet alcohol through reduction of the allyl alcohol.

In the elementary reactions (1) to (4), various investigations have been reported for the kind and the amount of the base catalyst used, the kind and the amount of the cocatalyst used, and the like, for such purposes as the suppression of the side reaction, the enhancement of the reaction rate, the enhancement of the yield and the enhancement of the quality of the Guerbet alcohol obtained, and the like.

For example, PTL 1 describes a method for producing a branched dimerized alcohol through reaction of an alcohol having 3 to 26 carbon atoms in the presence of (a) a catalyst formed of an alkaline substance and (b) a catalyst which is copper, a fourth period transition metal element (e.g., chromium, cobalt, nickel, manganese, iron, and zinc), and a Group 8 platinum group element (e.g., platinum, palladium, ruthenium, and rhodium).

Citation List

Patent Literature

PTL 1: JP 2-286638 A

Summary of the Invention

However, according to the method for producing a branched dimerized alcohol described in PTL 1, it is stated that the reaction time can be shortened, and the yield and the selectivity of the alcohol formed can be enhanced, but there is room for improvement from the standpoint of the suppression of the side reaction and the enhancement of the quality of the Guerbet alcohol.

Examples of the by-products of the elementary reactions (1) to (4) include an ester having a number of carbon atoms that is twice the raw material alcohol, i.e., the same number of carbon atoms as the Guerbet alcohol formed (which may be hereinafter referred to as a "by-produced ester"). The by-produced ester is difficult to remove through distillation since the by-produced ester has a similar boiling point as the Guerbet alcohol formed, and thus has been a problem.

Under the circumstances, a problem to be solved by the present invention is to provide a method for producing a Guerbet alcohol having a high quality with a less amount of impurities while suppressing the formation of the by-produced ester.

The present inventors have found that the problem can be solved by reacting a raw material alcohol having 8 or more and 22 or less carbon atoms in the presence of a particular catalyst (A).

The present invention relates to the following items [1] and [2].

[1] A method for producing a Guerbet alcohol, including reacting a raw material alcohol having 8 or more and 22 or less carbon atoms, in the presence of a catalyst (A) containing a first component, a second component, and a third component below:

first component: copper,

second component: one kind selected from the group consisting of cobalt, nickel, molybdenum, and rhenium, and third component: at least one kind selected from the group consisting of elements that are elements belonging to Groups 3 to 10 and 12 of the fourth period of the periodic table and elements belonging to Groups 3 to 7, 11, and 12 of the fifth and sixth periods of the periodic table, and are different from the element selected as the second component.

[2] A catalyst used for a method for producing a Guerbet alcohol, containing a first component, a second component, and a third component below:

first component: copper,
second component: one kind selected from the group consisting of cobalt, nickel, molybdenum, and rhenium, and third component: at least one kind selected from the group consisting of elements that are elements belonging to Groups 3 to 10 and 12 of the fourth period of the periodic table and elements belonging to Groups 3 to 7, 11, and 12 of the fifth and sixth periods of the periodic table, and are different from the element selected as the second component.

According to the present invention, a method for producing a Guerbet alcohol having a high quality with a less amount of impurities while suppressing the formation of the by-produced ester can be provided.

Detailed Description of the Invention

[Raw Material Alcohol]

In the method for producing a Guerbet alcohol of the present invention, an alcohol having 8 or more and 22 or less carbon atoms (which may be hereinafter referred simply to as a "raw material alcohol") is used.

The number of carbon atoms of the raw material alcohol is 8 or more, preferably 9 or more, and more preferably 10 or more, and is 22 or less, preferably 20 or less, and more preferably 18 or less, from the standpoint of the yield.

The raw material alcohol may be used alone or as a combination of two or more kinds thereof.

Examples of the raw material alcohol include a primary aliphatic alcohol and a secondary aliphatic alcohol, among which a primary aliphatic alcohol is preferred from the standpoint of the yield, among which a primary aliphatic alcohol having 8 or more and 18 or less carbon atoms is preferred, and a saturated linear primary aliphatic alcohol having 8 or more and 18 or less carbon atoms is more preferred.

Specific examples of the primary aliphatic alcohol include a saturated linear alcohol, such as 1-octanol (C8), 1-nonanol (C9), 1-decanol (C10), 1-undecanol (C11), 1-dodecanol (C12), 1-tridecanol (C13), 1-tetradecanol (C14), 1-pentadecanol (C15), 1-hexadecanol (C16), 1-heptadecanol (C17), 1-octadecanol (C18), 1-nonadecanol (C19), 1-eicosanol (C20), 1-heneicosanol (C21), and 1-docosanol (C22); a saturated alicyclic alcohol, such as cyclohexaneethanol (C8), cyclohexanepropanol (C9), and cyclohexanebutanol (C10); and an unsaturated alcohol, such as citronellol (C10) and oleyl alcohol (C18).

Specific examples of the secondary aliphatic alcohol include a saturated linear alcohol, such as 2-octanol (C8), 2-nonanol (C9), 2-decanol (C10), 2-undecanol (C11), 2-dodecanol (C12), 2-tridecanol (C13), 2-tetradecanol (C14), 2-pentadecanol (C15), 2-hexadecanol (C16), 2-heptadecanol (C17), 2-octadecanol (C18), 2-nonadecanol (C19), 2-eicosanol (C20), 2-heneicosanol (C21), and 2-docosanol (C22).

[Catalyst (A)]

In the method for producing a Guerbet alcohol of the present invention, the catalyst (A) containing the particular components is used, and the catalyst (A) that includes a carrier having the particular components supported thereon may also be used. The use of the catalyst (A) can suppress the formation of the by-produced ester and can provide a Guerbet alcohol having a high quality with a less amount of impurities.

The catalyst (A) used in the present invention is a catalyst containing the first component, the second component, and the third component shown below, and may also be a catalyst that includes a carrier having the first component, the second component, and the third component shown below supported thereon:

first component: copper,
second component: one kind selected from the group consisting of cobalt, nickel, molybdenum, and rhenium, and third component: at least one kind selected from the group consisting of elements that are elements belonging to Groups 3 to 10 and 12 of the fourth period of the periodic table and elements belonging to Groups 3 to 7, 11, and 12 of the fifth and sixth periods of the periodic table, and are different from the element selected as the second component.

(First Component)

The first component of the catalyst (A) is not particularly limited, as far as the component is copper (Cu), and an oxide thereof may also be used.

The content of the first component (Cu) contained in the catalyst (A) is preferably 0.5% by mass or more, more preferably 1% by mass or more, and further preferably 3% by mass or more, from the standpoint of the yield, and is preferably 45% by mass or less, more preferably 39% by mass or less, and further preferably 36% by mass or less, from the standpoint of the yield and the economic efficiency.

The content of the first component contained in the catalyst (A) can be obtained specifically through measurement in the manner described in the examples.

The average primary particle diameter of the first component (Cu) contained in the catalyst (A) is preferably 0.2 nm or more, more preferably 1 nm or more, and further preferably 3 nm or more, and is preferably 50 nm or less, more preferably 40 nm or less, and further preferably 30 nm or less, from the standpoint of the yield.

The average primary particle diameter of the first component (Cu) contained in the catalyst (A) can be obtained specifically through measurement in the manner described in the examples.

(Second Component)

The second component of the catalyst (A) is not particularly limited, as far as the component is one kind selected from the group consisting of cobalt (Co), nickel (Ni), molybdenum (Mo), and rhenium (Re), and an oxide thereof may also be used.

In the second component, one kind selected from the group consisting of cobalt (Co), nickel (Ni), and rhenium (Re) is preferred from the standpoint of the yield and the selectivity.

The content of the second component contained in the catalyst (A) is preferably 0.5% by mass or more, more preferably 1% by mass or more, and further preferably 2% by mass or more, from the standpoint of the selectivity, and is preferably 30% by mass or less, more preferably 25% by mass or less, and further preferably 20% by mass or less, from the standpoint of the yield.

The content of the second component contained in the catalyst (A) can be obtained specifically through measurement in the manner described in the examples.

(Third Component)

The third component of the catalyst (A) is not particularly limited, as far as the component is at least one kind selected from the group consisting of elements that are elements belonging to Groups 3 to 10 and 12 of the fourth period of the periodic table and elements belonging to Groups 3 to 7, 11, and 12 of the fifth and sixth periods of the periodic table, and are different from the element selected as the second component, and an oxide thereof may also be used.

Examples of the elements belonging to Groups 3 to 10 and 12 of the fourth period of the periodic table include scandium (Sc), titanium (Ti), vanadium (V), chromium (Cr), manganese (Mn), iron (Fe), cobalt (Co), nickel (Ni), and zinc (Zn).

Examples of the elements belonging to Groups 3 to 7, 11, and 12 of the fifth period of the periodic table include yttrium (Y), zirconium (Zr), niobium (Nb), molybdenum (Mo), technetium (Tc), silver (Ag), and cadmium (Cd).

Examples of the elements belonging to Groups 3 to 7, 11, and 12 of the sixth period of the periodic table include lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), hafnium (Hf), tantalum (Ta), tungsten (W), rhenium (Re), gold (Au), and mercury (Hg).

The third component is preferably at least one kind selected from the group consisting of titanium (Ti), iron (Fe), zinc (Zn), yttrium (Y), zirconium (Zr), niobium (Nb), molybdenum (Mo), lanthanum (La), cerium (Ce), samarium (Sm), tantalum (Ta), tungsten (W), rhenium (Re), and gold (Au) from the standpoint of the yield and the selectivity.

The third component is preferably at least one kind selected from the group consisting of elements belonging to Groups 3 to 7 of the fourth to sixth periods of the periodic table from the standpoint of the selectivity and/or the yield.

Examples of the elements belonging to Groups 3 to 7 of the fourth period of the periodic table include scandium (Sc), titanium (Ti), vanadium (V), chromium (Cr), and manganese (Mn).

Examples of the elements belonging to Groups 3 to 7 of the fifth period of the periodic table include yttrium (Y), zirconium (Zr), niobium (Nb), molybdenum (Mo), and technetium (Tc).

Examples of the elements belonging to Groups 3 to 7 of the sixth period of the periodic table include lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), hafnium (Hf), tantalum (Ta), tungsten (W), and rhenium (Re).

The third component is preferably at least one kind selected from the group consisting of titanium (Ti), yttrium (Y), niobium (Nb), molybdenum (Mo), lanthanum (La), cerium (Ce), samarium (Sm), tantalum (Ta), tungsten (W), and rhenium (Re) from the standpoint of the selectivity, among which at least one kind selected from the group consisting of titanium (Ti), yttrium (Y), molybdenum (Mo), lanthanum (La), cerium (Ce), samarium (Sm), tantalum (Ta), and rhenium (Re) is more preferred.

The third component is preferably at least one kind selected from the group consisting of titanium (Ti), yttrium (Y), zirconium (Zr), niobium (Nb), lanthanum (La), cerium (Ce), samarium (Sm), tantalum (Ta), and tungsten (W) from the standpoint of the yield.

The third component is preferably at least one kind selected from the group consisting of titanium (Ti), yttrium (Y), niobium (Nb), lanthanum (La), cerium (Ce), samarium (Sm), tantalum (Ta), and tungsten (W) from the standpoint of the yield and the selectivity.

The content of the third component contained in the catalyst (A) is preferably 0.1% by mass or more, more preferably 0.3% by mass or more, and further preferably 0.5% by mass or more, from the standpoint of the yield and the selectivity, and is preferably 15% by mass or less, more preferably 10% by mass or less, and further preferably 5% by mass or less, from the standpoint of the yield.

The content of the third component contained in the catalyst (A) can be obtained specifically through measurement in the manner described in the examples.

In the catalyst (A), the molar ratio of the second component to the first component is preferably 0.01 or more, more preferably 0.03 or more, further preferably 0.04 or more, still further preferably 0.05 or more, still more further preferably 0.13 or more, and yet more further preferably 0.16 or more, and is preferably 10 or less, more preferably 9 or less, further preferably 6 or less, still further preferably 5 or less, still more further preferably 2 or less, and yet more further preferably 1 or less, from the standpoint of the yield.

In the catalyst (A), the molar ratio of the third component to the first component is preferably 0.0003 or more, more preferably 0.001 or more, further preferably 0.003 or more, still further preferably 0.007 or more, and still more further preferably 0.01 or more, and is preferably 0.8 or less, more preferably 0.7 or less, further preferably 0.6 or less, and still further preferably 0.5 or less, from the standpoint of the yield.

The catalyst (A) may contain an element other than the first component, the second component, and the third component, in such a range that does not impair the effects of the present invention.

(Carrier)

The carrier of the catalyst (A) is not particularly limited, as far as the carrier can support the first component, the second component, and the third component.

Examples of the carrier of the catalyst (A) include a carbon material, such as activated carbon, nano carbon, and carbon black; and an inorganic material, such as aluminum oxide, iron oxide, copper oxide, titanium oxide, zirconium oxide, zeolite, cerium oxide, and hydrotalcite. Among these, the carrier of the catalyst (A) is preferably at least one kind selected from the group consisting of aluminum oxide, activated carbon, titanium oxide, zirconium oxide, zeolite, cerium oxide, and hydrotalcite from the standpoint of the versatility and the economic efficiency, among which at least one kind selected from the group consisting of aluminum oxide, zirconium oxide, and hydrotalcite is more preferred.

The shape of the carrier is not particularly limited, and is generally in the form of powder, the median diameter (d50) of which is generally 1 to 300 pm, and the shape thereof may be other shapes derived from powder depending on necessity.

The amount of the first to third components supported on the carrier (supported amount) in 100% by mass of the catalyst (A) is preferably 1% by mass or more, more preferably 3% by mass or more, and further preferably 5% by mass or more, from the standpoint of the yield, and is preferably 60% by mass or less, more preferably 50% by mass or less, and further preferably 45% by mass or less, from the standpoint of the yield and the economic efficiency.

The "amount of the first to third components supported on the carrier (supported amount) in 100% by mass of the catalyst (A)" herein means the total of the contents of the first to third components contained in the catalyst (A).

In the catalyst (A), the mass ratio of the second component to the first component is preferably 0.10 or more, more preferably 0.12 or more, and further preferably 0.15 or more, from the standpoint of the selectivity, and is preferably 9 or less, more preferably 5 or less, and further preferably 1 or less, from the standpoint of the yield.

In the catalyst (A), the mass ratio of the third component to the first component is preferably 0.001 or more, more preferably 0.005 or more, and further preferably 0.01 or more, from the standpoint of the yield and the selectivity, and is preferably 0.60 or less, more preferably 0.50 or less, and further preferably 0.40 or less, from the standpoint of the yield.

The shape of the catalyst (A) is not particularly limited, and examples thereof include powder, granules, noodle-like form, and pellets. The shapes including granules, noodle-like form, and pellets can be produced through granulation or molding the catalyst (A) in the form of powder by a known method.

In the case where the catalyst (A) is in the form of powder, the median diameter (d50) of the catalyst (A) is preferably 1 pm or more, more preferably 3 pm or more, further preferably 5 pm or more, and still further preferably 7 pm or more, from the standpoint of the recoverability, and is preferably 300 pm or less, more preferably 200 pm or less, further preferably 100 pm or less, and still further preferably 30 pm or less, from the standpoint of the yield. The median diameter (d50) of the catalyst (A) can be measured with a laser diffraction/scattering particle size distribution analyzer "LA-920" (produced by Horiba, Ltd.). The measurement may be performed after dispersing in 0.05 g of ion exchanged water as the measurement solvent under stirring (stirring rate: level 4), and the median diameter (d50) is calculated with an appropriate relative refractive index.

In the case where the catalyst (A) is in the form of granules, the average particle diameter of the catalyst (A) is preferably 0.2 mm or more, more preferably 0.4 mm or more, and further preferably 0.6 mm or more, from the standpoint of the recoverability, and is preferably 2.0 mm or less, more preferably 1.3 mm or less, and further preferably 0.8 mm or less, from the standpoint of the yield. The average particle diameter of the catalyst (A) herein means the arithmetic average particle diameter, and can be obtained with a vernier caliper. The number of the granules for obtaining the average particle diameter may be 30 granules randomly selected.

In the case where the catalyst (A) is in the noodle-like form, the average diameter of the catalyst (A) is preferably 1.0 mm or more, more preferably 1.2 mm or more, and further preferably 1.4 mm or more, from the standpoint of the strength of the catalyst, and is preferably 2.5 mm or less, more preferably 2.0 mm or less, and further preferably 1.5 mm or less, from the standpoint of the yield. The average diameter of the catalyst (A) herein means the arithmetic average diameter, and can be obtained with a vernier caliper. The number of the noodles for obtaining the average diameter may be 30 noodles randomly selected.

In the case where the catalyst (A) is in the noodle-like form, the average length of the catalyst (A) is preferably 2 mm or more, and more preferably 3 mm or more, from the standpoint of the strength of the catalyst, and is preferably 8 mm or less, more preferably 6 mm or less, and further preferably 4 mm or less, from the standpoint of the homogeneity in packing and the yield. The average length of the catalyst (A) herein means the arithmetic average length, and can be obtained with a vernier caliper. The number of the noodles for obtaining the average length may be 30 noodles randomly selected.

In the case where the catalyst (A) is in the form of pellets, the average diameter and the average height of the catalyst (A) each are preferably 1.5 mm or more, more preferably 2.0 mm or more, and further preferably 2.5 mm or more, from the standpoint of the strength of the catalyst, and is preferably 5.0 mm or less, more preferably 4.0 mm or less, and further preferably 3.0 mm or less, from the standpoint of the yield. The average diameter and the average height of the catalyst (A) herein mean the arithmetic average diameter and the arithmetic average height respectively, and can be obtained with a vernier caliper. The number of the pellets for obtaining the average diameter or the average height may be 30 noodles randomly selected.

(Preparation of Catalyst (A))

The catalyst (A) used in the present invention may be prepared by a known method, such as a precipitation method, an impregnation method, an ion exchange method, an alloying method, and an adsorption method.

The catalyst (A) may be prepared preferably by a method of supporting the first component and the second component by the precipitation method on the carrier, and then supporting the third component by the impregnation method on the carrier having the first component and the second component supported thereon.

The precipitation method for supporting the first component and the second component on the carrier may be performed, for example, in the following manner.

A first component-containing water-soluble salt and a second component-containing water-soluble salt are dissolved in ion exchanged water to prepare a mixed aqueous solution containing the first component and the second component. Separately, an alkali aqueous solution containing an alkali component, such as sodium carbonate, and a slurry containing a component to be the carrier, such as zirconium oxide, are prepared, respectively. Subsequently, the mixed aqueous solution containing the first component and the second component is added dropwise to the slurry, simultaneously the alkali aqueous solution is added dropwise thereto, and the solutions are added dropwise thereto for a prescribed period of time while retaining a prescribed pH of the slurry for insolubilizing and depositing the first component and the second component as a carbonate salt or a hydroxide, so as to provide a solid matter including the carrier having the carbonate salt or the hydroxide of the first component and the second component attached thereto. The solid matter is repeatedly subjected to an operation including filtration and washing, and baked at a prescribed temperature for a prescribed period of time, so as to provide a baked material including the carrier having the first component and the second component supported thereon.

The impregnation method for supporting the third component on the carrier having the first component and the second component supported thereon may be performed, for example, in the following manner.

A third component-containing compound is dissolved in an organic solvent or water, to which the baked material of the solid matter including the carrier having the first component and the second component supported thereon is added, and the mixture is concentrated until the liquid phase disappears, for example, through depressurization or heating under stirring, then dried at a prescribed temperature, and further baked at a prescribed temperature for a prescribed period of time, so as to provide the catalyst (A) as a baked material including the carrier having the first to third components supported thereon.

The baking temperature for providing the baked material including the carrier having the first and second components supported thereon and the baked material including the carrier having the first to third components supported thereon is preferably 300°C or more, more preferably 350°C or more, and further preferably 400°C or more, and is preferably 900°C or less, more preferably 850°C or less, and further preferably 800°C or less, from the standpoint of the yield of the Guerbet alcohol.

The baking time for providing the baked material including the carrier having the first and second components supported thereon and the baked material including the carrier having the first to third components supported thereon is preferably 1 hour or more, more preferably 2 hours or more, and further preferably 3 hours or more, and is preferably 10 hours or less, more preferably 7 hours or less, and further preferably 5 hours or less, from the standpoint of the yield of the Guerbet alcohol.

The baking atmosphere for providing the baked material including the carrier having the first and second components supported thereon and the baked material including the carrier having the first to third components supported thereon is not particularly limited, examples of which include an inert gas atmosphere, such as nitrogen, an oxidizing atmosphere, such as air, and a reducing atmosphere, such as hydrogen, and among these, an oxidizing atmosphere, such as air, is preferred from the standpoint of the yield of the Guerbet alcohol. The baking atmosphere may be either a close state or an open state.

[Base Catalyst (B)]

In the method for producing a Guerbet alcohol of the present invention, a base catalyst (B) is preferably used with the catalyst (A).

The use of the base catalyst (B) with the catalyst (A) can facilitate the suppression of the formation of the by-produced ester and can facilitate the production of a Guerbet alcohol having a high quality with a less amount of impurities.

Examples of the base catalyst (B) include an alkali metal and an alkaline earth metal, and hydrides, hydroxides, carbonates, hydrogen carbonates, and alkoxides thereof.

Specific examples of hydrides, hydroxides, carbonates, hydrogen carbonates, and alkoxide compounds of an alkali metal and an alkaline earth metal include an alkali metal hydroxide, such as LiOH, NaOH, KOH, RbOH, and CsOH; an alkali metal carbonate, such as $Li_2CO_3$, $Na_2CO_3$, $K_2CO_3$, $Rb_2CO_3$, $CS_2CO_3$; an alkali metal hydrogen carbonate, such as $LiHCO_3$, $NaHCO_3$, $KHCO_3$, $RbHCO_3$, $CsHCO_3$; an alkali metal alkoxide compound, such as sodium methoxide, sodium ethoxide, sodium t-butoxide, potassium methoxide, potassium ethoxide, and potassium t-butoxide; and an alkaline earth metal hydroxide, such as $Mg(OH)_2$ and $Ca(OH)_2$.

In the base catalyst (B), an alkali metal hydroxide, such as LiOH, NaOH, KOH, RbOH, and CsOH; and an alkali metal alkoxide compound, such as sodium methoxide, sodium ethoxide, sodium t-butoxide, potassium methoxide, potassium ethoxide, and potassium t-butoxide, all of which are strong bases, are preferred from the standpoint of the yield, among which NaOH and KOH are more preferred from the standpoint of the versatility and the economic efficiency.

The base catalyst (B) may be used alone or as a combination of two or more kinds thereof.

The base catalyst (B) may not be supported on a carrier.

The amount of the base catalyst (B) based on the total amount of the raw material alcohol is preferably 0.05% by mol or more, more preferably 0.1% by mol or more, and further preferably 0.2% by mol or more, from the standpoint of the yield, and is preferably 7% by mol or less, more preferably 5% by mol or less, and further preferably 3% by mol or less, from the standpoint of the selectivity.

[Guerbet Reaction]

In the method for producing a Guerbet alcohol of the present invention, a raw material alcohol having 8 or more and 22 or less carbon atoms is reacted (dehydration condensation reaction (Guerbet reaction)) in the presence of the catalyst (A) containing the particular components and/or the catalyst (A) including the carrier having the particular components supported thereon, so as to form a Guerbet alcohol.

The reaction mode of the Guerbet reaction is not particularly limited, and may be either suspended bed reaction or fixed bed reaction, which may be appropriately selected depending on the catalyst activity, the reaction scale, and the like. The material of the reaction equipment used for the Guerbet reaction may be a stainless steel (such as SUS201, SUS202, SUS301, SUS302, SUS303, SUS304, SUS305, SUS316, SUS317, SUS329J1, SUS403, SUS405, SUS420, SUS430, SUS430LX, and SUS630), and may be glass.

The method for producing a Guerbet alcohol of the present invention may be either a batch system, a semi-batch system, or a continuous system.

In the case where the reaction mode of the reaction is suspended bed reaction, a batch system or a semi-batch system is preferred from the standpoint of the operability, and the amount of the catalyst (A) based on the total amount of the raw material alcohol is preferably 0.01% by mass or more, more preferably 0.05% by mass or more, and further preferably 0.1% by mass or more, from the standpoint of the yield, and is preferably 10% by mass or less, more preferably 5% by mass or less, and further preferably 1% by mass or less, from the standpoint of the economic efficiency.

In the case where the reaction mode of the reaction is fixed bed reaction, a continuous system is preferred from the standpoint of the productivity, and the amount of the catalyst (A) based on the total amount of the raw material alcohol is preferably 10% by mass or more, more preferably 15% by mass or more, further preferably 25% by mass or more, and still further preferably 50% by mass or more, from the standpoint of the yield, and is preferably 4,000% by mass or less, more preferably 2,500% by mass or less, further preferably 1,000% by mass or less, and still further preferably 500% by mass or less, from the standpoint of the economic efficiency.

The reaction temperature of the Guerbet reaction may be appropriately determined in consideration of the boiling point of the raw material alcohol, is preferably 180°C or more, more preferably 190°C or more, further preferably 200°C or more, and still further preferably 220°C or more, from the standpoint of the yield, and is preferably 300°C or less, more preferably 280°C or less, and further preferably 260°C or less, from the standpoint of the selectivity.

The reaction time of the Guerbet reaction may be appropriately determined depending on the reaction temperature and the kind of the raw material alcohol, and the reaction time in the suspended bed reaction is generally 1 hour or more from the standpoint of the yield, and is preferably 20 hours or less, and more preferably 10 hours or less, from the standpoint of the productivity. The LHSV (liquid hourly space velocity) in the fixed bed reaction is preferably 10 $hr^{-1}$ or less, more preferably 7 $hr^{-1}$ or less, further preferably 5 $hr^{-1}$ or less, and still further preferably 3 $hr^{-1}$ or less, from the standpoint of the yield, and is preferably 0.03 $hr^{-1}$ or more, more preferably 0.05 $hr^{-1}$ or more, further preferably 0.1 $hr^{-1}$ or more, and still further preferably 0.2 $hr^{-1}$ or more, from the standpoint of the productivity.

The pressure of the gas phase in reaction of the Guerbet reaction may be either reduced pressure, ordinary pressure, or increased pressure, may be reduced pressure from the standpoint of the yield and the selectivity, and may be ordinary pressure from the standpoint of the operability and the production cost.

In the Guerbet reaction, it is preferred from the standpoint of the yield and the selectivity that an inert gas is introduced to the reaction system, and the inert gas is allowed to flow as a carrier gas. The inert gas is not particularly limited, examples of which include nitrogen gas, argon gas, and carbon dioxide gas, and among these, nitrogen gas is preferably used.

The inert gas may be allowed to flow by a method of flowing in the upper part of the reaction liquid, a method of bubbling in the reaction liquid, and the like.

The flow rate of the inert gas in increasing the temperature until reaching the reaction temperature is not particularly limited, and the flow rate per 1 kg of the reaction liquid is preferably 0.5 L/hr or more, more preferably 3 L/hr or more, and further preferably 8 L/hr or more, from the standpoint of the yield and the selectivity, and is preferably 30 L/hr or less, more preferably 25 L/hr or less, and further preferably 20 L/hr or less, from the standpoint of the economic efficiency.

The flow rate of the inert gas in the reaction after reaching the reaction temperature is not particularly limited, and the flow rate per 1 kg of the reaction liquid is preferably 0.02 L/hr or more, more preferably 0.08 L/hr or more, and further preferably 0.1 L/hr or more, from the standpoint of the yield and the selectivity, and is preferably 10 L/hr or less, more preferably 5 L/hr or less, and further preferably 2 L/hr or less, from the standpoint of the economic efficiency.

The Guerbet alcohol formed in the production method of the present invention is determined depending on the kind of the raw material alcohol used, may be saturated or unsaturated, may be primary or secondary, and may have a cyclic structure.

The number of carbon atoms of the Guerbet alcohol formed in the production method of the present invention is preferably 16 or more, more preferably 18 or more, and further preferably 20 or more, and is preferably 44 or less, more preferably 40 or less, and further preferably 36 or less, from the standpoint of the yield.

The production method of the present invention is excellent in effect of suppressing the formation of the by-produced ester (the dimer ester) with a low by-production rate of the by-produced ester (the dimer ester), and can provide a Guerbet alcohol having a high quality with a less amount of impurities. Accordingly, the Guerbet alcohol obtained by the production method of the present invention can be applied directly to various purposes, and may also be applied after purification by a distillation operation or the like depending on necessity. A Guerbet alcohol is useful as a raw material or an intermediate material of surfactants, textile oil agents, fabric softeners, cosmetics, medical drugs, lubricating oils, and the like. From the standpoint of the application to these purposes, the purity of the Guerbet alcohol is preferably 95% by mass or more, more preferably 97% by mass or more, and further preferably 98% by mass or more.

In addition to the aforementioned embodiments, the present invention relates to the following methods for producing a Guerbet alcohol.

<1> A method for producing a Guerbet alcohol, including reacting a raw material alcohol having 8 or more and 22 or less carbon atoms, in the presence of a catalyst (A) containing a first component, a second component, and a third component below:

first component: copper (Cu),
second component: one kind selected from the group consisting of cobalt (Co), nickel (Ni), molybdenum (Mo), and rhenium (Re), and
third component: at least one kind selected from the group consisting of elements that are elements belonging to Groups 3 to 10 and 12 of the fourth period of the periodic table and elements belonging to Groups 3 to 7, 11, and 12 of the fifth and sixth periods of the periodic table, and are different from the element selected as the second component.

<2> The method for producing a Guerbet alcohol according to the item <1>, wherein the number of carbon atoms of the raw material alcohol is preferably 9 or more, and more preferably 10 or more, and is preferably 20 or less, and more preferably 18 or less.
<3> The method for producing a Guerbet alcohol according to the item <1> or <2>, wherein the number of carbon atoms of the raw material alcohol is preferably 8 or more and 20 or less.
<4> The method for producing a Guerbet alcohol according to the item <1> or <2>, wherein the number of carbon atoms of the raw material alcohol is preferably 8 or more and 18 or less.
<5> The method for producing a Guerbet alcohol according to the item <1> or <2>, wherein the number of carbon atoms of the raw material alcohol is preferably 9 or more and 20 or less.
<6> The method for producing a Guerbet alcohol according to the item <1> or <2>, wherein the number of carbon atoms of the raw material alcohol is preferably 9 or more and 18 or less.
<7> The method for producing a Guerbet alcohol according to the item <1> or <2>, wherein the number of carbon atoms of the raw material alcohol is preferably 10 or more and 20 or less.
<8> The method for producing a Guerbet alcohol according to the item <1> or <2>, wherein the number of carbon atoms of the raw material alcohol is preferably 10 or more and 18 or less.
<9> The method for producing a Guerbet alcohol according to the item <1> or <2>, wherein the raw material alcohol is preferably a primary aliphatic alcohol.
<10> The method for producing a Guerbet alcohol according to the item <1> or <2>, wherein the raw material alcohol is preferably a primary aliphatic alcohol having 8 or more and 18 or less carbon atoms.
<11> The method for producing a Guerbet alcohol according to the item <1> or <2>, wherein the raw material alcohol is preferably a saturated linear primary aliphatic alcohol having 8 or more and 18 or less carbon atoms.
<12> The method for producing a Guerbet alcohol according to any one of the items <1> to <11>, wherein the content of the first component contained in the catalyst (A) is preferably 0.5% by mass or more, more preferably 1% by mass or more, and further preferably 3% by mass or more, and is preferably 45% by mass or less, more preferably 39% by mass or less, and further preferably 36% by mass or less.
<13> The method for producing a Guerbet alcohol according to any one of the items <1> to <12>, wherein the average primary particle diameter of the first component contained in the catalyst (A) is preferably 0.2 nm or more, more preferably 1 nm or more, and further preferably 3 nm or more, and is preferably 50 nm or less, more preferably 40 nm or less, and further preferably 30 nm or less.
<14> The method for producing a Guerbet alcohol according to any one of the items <1> to <13>, wherein the second component is preferably one kind selected from the group consisting of cobalt (Co), nickel (Ni), and rhenium (Re).
<15> The method for producing a Guerbet alcohol according to any one of the items <1> to <14>, wherein the content of the second component contained in the catalyst (A) is preferably 0.5% by mass or more, more preferably 1% by mass or more, and further preferably 2% by mass or more, and is preferably 30% by mass or less, more preferably 25% by mass or less, and further preferably 20% by mass or less.
<16> The method for producing a Guerbet alcohol according to any one of the items <1> to <15>, wherein the third component is preferably at least one kind selected from the group consisting of titanium (Ti), iron (Fe), zinc (Zn), yttrium (Y), zirconium (Zr), niobium (Nb), molybdenum (Mo), lanthanum (La), cerium (Ce), samarium (Sm), tantalum (Ta), tungsten (W), rhenium (Re), and gold (Au).
<17> The method for producing a Guerbet alcohol according to any one of the items <1> to <15>, wherein the third component is preferably at least one kind selected from the group consisting of elements belonging to Groups 3 to 7 of the fourth to sixth periods of the periodic table.

<18> The method for producing a Guerbet alcohol according to the item <17>, wherein the third component is preferably at least one kind selected from the group consisting of titanium (Ti), yttrium (Y), niobium (Nb), molybdenum (Mo), lanthanum (La), cerium (Ce), samarium (Sm), tantalum (Ta), tungsten (W), and rhenium (Re), among which at least one kind selected from the group consisting of titanium (Ti), yttrium (Y), molybdenum (Mo), lanthanum (La), cerium (Ce), samarium (Sm), tantalum (Ta), and rhenium (Re) is more preferred.

<19> The method for producing a Guerbet alcohol according to the item <17>, wherein the third component is preferably at least one kind selected from the group consisting of titanium (Ti), yttrium (Y), zirconium (Zr), niobium (Nb), lanthanum (La), cerium (Ce), samarium (Sm), tantalum (Ta), and tungsten (W).

<20> The method for producing a Guerbet alcohol according to the item <17>, wherein the third component is preferably at least one kind selected from the group consisting of titanium (Ti), yttrium (Y), niobium (Nb), lanthanum (La), cerium (Ce), samarium (Sm), tantalum (Ta), and tungsten (W).

<21> The method for producing a Guerbet alcohol according to any one of the items <1> to <20>, wherein the content of the third component contained in the catalyst (A) is preferably 0.1% by mass or more, more preferably 0.3% by mass or more, and further preferably 0.5% by mass or more, and is preferably 15% by mass or less, more preferably 10% by mass or less, and further preferably 5% by mass or less.

<22> The method for producing a Guerbet alcohol according to any one of the items <1> to <21>, wherein in the catalyst (A), the molar ratio of the second component to the first component is preferably 0.01 or more, more preferably 0.03 or more, further preferably 0.04 or more, still further preferably 0.05 or more, still more further preferably 0.13 or more, and yet more further preferably 0.16 or more, and is preferably 10 or less, more preferably 9 or less, further preferably 6 or less, still further preferably 5 or less, still more further preferably 2 or less, and yet more further preferably 1 or less.

<23> The method for producing a Guerbet alcohol according to any one of the items <1> to <22>, wherein in the catalyst (A), the molar ratio of the third component to the first component is preferably 0.0003 or more, more preferably 0.001 or more, further preferably 0.003 or more, still further preferably 0.007 or more, and still more further preferably 0.01 or more, and is preferably 0.8 or less, more preferably 0.7 or less, further preferably 0.6 or less, and still further preferably 0.5 or less.

<24> The method for producing a Guerbet alcohol according to any one of the items <1> to <23>, wherein the catalyst (A) is a catalyst including a carrier having the first component, the second component, and the third component supported thereon, and the carrier is preferably at least one kind selected from the group consisting of aluminum oxide, activated carbon, titanium oxide, zirconium oxide, zeolite, cerium oxide, and hydrotalcite, among which at least one kind selected from the group consisting of aluminum oxide, zirconium oxide, and hydrotalcite is more preferred.

<25> The method for producing a Guerbet alcohol according to the item <24>, wherein the amount of the first to third components supported on the carrier (supported amount) in 100% by mass of the catalyst (A) is preferably 1% by mass or more, more preferably 3% by mass or more, and further preferably 5% by mass or more, and is preferably 60% by mass or less, more preferably 50% by mass or less, and further preferably 45% by mass or less.

<26> The method for producing a Guerbet alcohol according to any one of the items <1> to <25>, wherein the mass ratio of the second component to the first component in the catalyst (A), is preferably 0.10 or more, more preferably 0.12 or more, and further preferably 0.15 or more, and is preferably 9 or less, more preferably 5 or less, and further preferably 1 or less.

<27> The method for producing a Guerbet alcohol according to any one of the items <1> to <26>, wherein the mass ratio of the third component to the first component in the catalyst (A) is preferably 0.001 or more, more preferably 0.005 or more, and further preferably 0.01 or more, and is preferably 0.60 or less, more preferably 0.50 or less, and further preferably 0.40 or less.

<28> The method for producing a Guerbet alcohol according to any one of the items <1> to <27>, wherein in the case where the catalyst (A) is in the form of powder, the median diameter (d50) of the catalyst (A) is preferably 1 pm or more, more preferably 3 pm or more, further preferably 5 pm or more, and still further preferably 7 pm or more, and is preferably 300 pm or less, more preferably 200 pm or less, further preferably 100 pm or less, and still further preferably 30 μm or less.

<29> The method for producing a Guerbet alcohol according to any one of the items <1> to <27>, wherein in the case where the catalyst (A) is in the form of granules, the average particle diameter of the catalyst (A) is preferably 0.2 mm or more, more preferably 0.4 mm or more, and further preferably 0.6 mm or more, and is preferably 2.0 mm or less, more preferably 1.3 mm or less, and further preferably 0.8 mm or less.

<30> The method for producing a Guerbet alcohol according to any one of the items <1> to <27>, wherein in the case where the catalyst (A) is in the noodle-like form, the average diameter of the catalyst (A) is preferably 1.0 mm or more, more preferably 1.2 mm or more, and further preferably 1.4 mm or more, and is preferably 2.5 mm or less, more preferably 2.0 mm or less, and further preferably 1.5 mm or less.

<31> The method for producing a Guerbet alcohol according to any one of the items <1> to <27>, wherein in the case where the catalyst (A) is in the noodle-like form, the average length of the catalyst (A) is preferably 2 mm or more, and more preferably 3 mm or more, and is preferably 8 mm or less, more preferably 6 mm or less, and further preferably 4 mm or less.

<32> The method for producing a Guerbet alcohol according to any one of the items <1> to <27>, wherein in the case where the catalyst (A) is in the form of pellets, the average diameter and the average height of the catalyst (A) each are preferably 1.5 mm or more, more preferably 2.0 mm or more, and further preferably 2.5 mm or more, and is preferably 5.0 mm or less, more preferably 4.0 mm or less, and further preferably 3.0 mm or less.

<33> The method for producing a Guerbet alcohol according to any one of the items <24> to <32>, wherein the baking temperature for providing the baked material including the carrier having the first and second components supported thereon and the baked material including the carrier having the first to third components supported thereon is preferably 300°C or more, more preferably 350°C or more, and further preferably 400°C or more, and is preferably 900°C or less, more preferably 850°C or less, and further preferably 800°C or less.

<34> The method for producing a Guerbet alcohol according to any one of the items <24> to <33>, wherein the baking time for providing the baked material including the carrier having the first and second components supported thereon and the baked material including the carrier having the first to third components supported thereon is preferably 1 hour or more, more preferably 2 hours or more, and further preferably 3 hours or more, and is preferably 10 hours or less, more preferably 7 hours or less, and further preferably 5 hours or less.

<35> The method for producing a Guerbet alcohol according to any one of the items <24> to <34>, wherein the baking atmosphere for providing the baked material including the carrier having the first and second components supported thereon and the baked material including the carrier having the first to third components supported thereon is preferably an oxidizing atmosphere.

<36> The method for producing a Guerbet alcohol according to any one of the items <1> to <35>, wherein a base catalyst (B) is used with the catalyst (A), and the base catalyst (B) is preferably an alkali metal hydroxide, such as LiOH, NaOH, KOH, RbOH, and CsOH; and an alkali metal alkoxide compound, such as sodium methoxide, sodium ethoxide, sodium t-butoxide, potassium methoxide, potassium ethoxide, and potassium t-butoxide, all of which are strong bases, among which NaOH and KOH are more preferred.

<37> The method for producing a Guerbet alcohol according to the item <36>, wherein the base catalyst (B) may not be supported on a carrier.

<38> The method for producing a Guerbet alcohol according to any one of the items <1> to <37>, wherein the amount of the base catalyst (B) based on the total amount of the raw material alcohol is preferably 0.05% by mol or more, more preferably 0.1% by mol or more, and further preferably 0.2% by mol or more, and is preferably 7% by mol or less, more preferably 5% by mol or less, and further preferably 3% by mol or less.

<39> The method for producing a Guerbet alcohol according to any one of the items <1> to <38>, wherein in the case where the reaction mode of the reaction is suspended bed reaction, a batch system or a semi-batch system is preferred, and the amount of the catalyst (A) based on the total amount of the raw material alcohol is preferably 0.01% by mass or more, more preferably 0.05% by mass or more, and further preferably 0.1% by mass or more, and is preferably 10% by mass or less, more preferably 5% by mass or less, and further preferably 1% by mass or less.

<40> The method for producing a Guerbet alcohol according to any one of the items <1> to <38>, wherein in the case where the reaction mode of the reaction is fixed bed reaction, a continuous system is preferred, and the amount of the catalyst (A) based on the total amount of the raw material alcohol is preferably 10% by mass or more, more preferably 15% by mass or more, further preferably 25% by mass or more, and still further preferably 50% by mass or more, and is preferably 4,000% by mass or less, more preferably 2,500% by mass or less, further preferably 1,000% by mass or less, and still further preferably 500% by mass or less.

<41> The method for producing a Guerbet alcohol according to any one of the items <1> to <40>, wherein the reaction temperature is preferably 180°C or more, more preferably 190°C or more, further preferably 200°C or more, and still further preferably 220°C or more, and is preferably 300°C or less, more preferably 280°C or less, and further preferably 260°C or less.

<42> The method for producing a Guerbet alcohol according to any one of the items <1> to <41>, wherein the reaction time in suspended bed reaction is preferably 20 hours or less, and more preferably 10 hours or less, and the LHSV (liquid hourly space velocity) in fixed bed reaction is preferably 10 $hr^{-1}$ or less, more preferably 7 $hr^{-1}$ or less, further preferably 5 $hr^{-1}$ or less, and still further preferably 3 $hr^{-1}$ or less, and is preferably 0.03 $hr^{-1}$ or more, more preferably 0.05 $hr^{-1}$ or more, further preferably 0.1 $hr^{-1}$ or more, and still further preferably 0.2 $hr^{-1}$ or more.

<43> The method for producing a Guerbet alcohol according to any one of the items <1> to <42>, wherein in the reaction, it is preferred that an inert gas is introduced to the reaction system, and the inert gas is allowed to flow as a carrier gas.

<44> The method for producing a Guerbet alcohol according to the item <43>, wherein the inert gas is nitrogen gas.

<45> The method for producing a Guerbet alcohol according to the item <43> or <44>, wherein the flow rate of the inert gas per 1 kg of the reaction liquid in increasing the temperature until reaching the reaction temperature is preferably 0.5 L/hr or more, more preferably 3 L/hr or more, and further preferably 8 L/hr or more, and is preferably 30 L/hr or less, more preferably 25 L/hr or less, and further preferably 20 L/hr or less.

<46> The method for producing a Guerbet alcohol according to any one of the items <43> to <45>, wherein the flow rate of the inert gas per 1 kg of the reaction liquid in the reaction after reaching the reaction temperature is preferably 0.02 L/hr or more, more preferably 0.08 L/hr or more, and further preferably 0.1 L/hr or more, and is preferably 10 L/hr or less, more preferably 5 L/hr or less, and further preferably 2 L/hr or less.

<47> The method for producing a Guerbet alcohol according to any one of the items <1> to <46>, wherein the number of carbon atoms of the Guerbet alcohol is preferably 16 or more, more preferably 18 or more, and further preferably 20 or more, and is preferably 44 or less, more preferably 40 or less, and further preferably 36 or less.

<48> The method for producing a Guerbet alcohol according to any one of the items <1> to <47>, wherein the purity of the Guerbet alcohol is preferably 95% by mass or more, more preferably 97% by mass or more, and further preferably 98% by mass or more.

Examples

The present invention will be described in more detail with reference to examples, but the present invention is not limited thereto. The measurements and evaluations in Preparation Examples, Comparative Preparation Examples, Examples, and Comparative Examples were performed in the following manner.

(1) Measurement by ICP Emission Spectroscopy

The elements contained in the catalysts obtained in Preparation Examples and Comparative Preparation Examples were quantitatively determined by the ICP emission spectroscopy (high frequency inductively coupled plasma emission spectroscopy: ICP-AES, ICP-OES) with an ICP emission spectral analyzer (product name: iCAP 6500 Duo, produced by Thermo Fisher Scientific, Inc.).

(2) Measurement of Average Primary Particle Diameter of Cu

The average primary particle diameter of Cu supported on the carrier contained in the catalysts obtained in Preparation Examples and Comparative Preparation Examples was measured by the pulse method with a catalyst analyzer (product name: BELCAT-B, produced by Nippon Bell Co., Ltd.). After reducing CuO under a 5% $H_2$/Ar gas stream at 150°C for 4 hours as the pretreatment, 5%$N_2$O/He gas was introduced at 50°C until reaching saturation, and the average primary particle diameter of Cu was measured from the total gas consumption amount.

(3) Measurement of Conversion of Raw Material Alcohol and By-Production Rate of Dimer Ester

In Examples and Comparative Examples, the solution after completing the reaction was diluted with hexane, and then the products were quantitatively determined by analyzing by gas chromatography (column: Ultra ALLOY-1 (MS/HT) capillary column 30.0 m × 250 pm (Frontier Laboratories, Ltd.), detector: FID, injection temperature: 300°C, detector temperature: 300°C, He flow rate: 4.6 mL/min) .

The conversion of the raw material alcohol and the by-production rate of the dimer ester were calculated from the result of gas chromatography according to the following expressions, respectively. The results are shown in Tables 1 to 4.

$$\text{Conversion of raw material alcohol (\%)} = 100 - (\text{residual amount of raw}$$
$$\text{material alcohol (mol)/charged amount of raw material alcohol (mol))} \times 100$$

$$\text{By-production rate of dimer ester (\%)} = ((\text{formation amount of dimer ester}$$
$$\text{(mol)} \times 2)/\text{charged amount of raw material alcohol (mol))} \times 100$$

The dimer ester in the case where 1-dodecanol (C12) is used as the raw material alcohol is a C24 ester, the dimer ester in the case where 1-decanol (C10) is used is a C20 ester, and the dimer ester in the case where 1-octadecanol (C18) is used is a C36 ester.

(Preparation Example A)

<Preparation of CuNi/ZrO$_2$ by Precipitation Method>

50 g of cupric nitrate trihydrate (produced by Kanto Chemical Co., Inc.) and 13 g of nickel nitrate hexahydrate (produced by Kanto Chemical Co., Inc.) were placed in a 300 mL beaker, to which 260 g of ion exchanged water was added for dissolving to prepare a mixed aqueous solution of cupric nitrate and nickel nitrate having a ratio Cu/Ni = 5/1 (mass ratio). Subsequently, 48 g of sodium carbonate (produced by Fujifilm Wako Pure Chemical Corporation) was placed in another 300 mL beaker, to which 269 g of ion exchanged water was added for dissolving to prepare a sodium carbonate aqueous solution. And further, 50 g of zirconium oxide (product name: RC-100 zirconium oxide, produced by Daiich Kigenso Kagaku Kyogyo Co., Ltd. (white powder, median diameter (d50): 1.5 to 4 $\mu$m)) was placed in another 2 L beaker, to which 875 g of ion exchanged water was added to prepare a slurry of zirconium oxide.

The cupric nitrate-nickel nitrate mixed aqueous solution was added dropwise to the slurry of zirconium oxide, and simultaneously the sodium carbonate aqueous solution was added dropwise thereto, while retaining the pH to 7 (35°C) over 80 minutes. After completing the dropwise addition, a solid matter including zirconium oxide to be a carrier having a carbonate or a hydroxide of Cu and Ni attached thereto was filtrated under reduced pressure, and the resulting cake was washed with 1 L of ion exchanged water. The cake was subjected four times to an operation including re-slurrying, filtration under reduced pressure, and washing with water, then dried at 120°C for 18 hours, and further baked in air at 500°C for 3 hours, so as to provide a CuNi/ZrO$_2$ baked material.

(Preparation Example 1)

<Preparation of CuNiTi/ZrO$_2$ by Impregnation Method>

0.36 g of titanium tetraisopropoxide (produced by Fujifilm Wako Pure Chemical Corporation) was placed in a 50 mL recovery flask, to which 4.7 g of 2-propanol (produced by Fujifilm Wako Pure Chemical Corporation) was added for completely dissolving. 2.0 g of the CuNi/ZrO$_2$ baked material obtained in Preparation Example A was added thereto, and the mixture was concentrated until the liquid phase disappeared with a rotary evaporator, then dried at 120°C for 18 hours, and further baked in air at 500°C for 3 hours, so as to provide a CuNiTi/ZrO$_2$ baked material (powder) as the catalyst (A).

The average primary particle diameter of Cu contained in the resulting baked material was 21 nm.

(Preparation Example 2)

<Preparation of CuNiZn/ZrO$_2$ by Impregnation Method>

The same procedure as in Preparation Example 1 was performed except that in the preparation of CuNiTi/ZrO$_2$ by the impregnation method in Preparation Example 1, 0.36 g of titanium tetraisopropoxide (produced by Fujifilm Wako Pure Chemical Corporation) was changed to 0.27 g of zinc nitrate hexahydrate (produced by Fujifilm Wako Pure Chemical Corporation), and the solvent was changed from 4.7 g of 2-propanol (produced by Fujifilm Wako Pure Chemical Corporation) to 6 g of ion exchanged water, so as to provide a CuNiZn/ZrO$_2$ baked material (powder) as the catalyst (A).

(Preparation Example 3)

<Preparation of CuNiY/ZrO$_2$ by Impregnation Method>

The same procedure as in Preparation Example 2 was performed except that in the preparation of CuNiZn/ZrO$_2$ by the impregnation method in Preparation Example 2, 0.27 g of zinc nitrate hexahydrate (produced by Fujifilm Wako Pure Chemical Corporation) was changed to 0.19 g of yttrium nitrate n-hydrate (produced by Fujifilm Wako Pure Chemical Corporation), so as to provide a CuNiY/ZrO$_2$ baked material (powder) as the catalyst (A).

The average primary particle diameter of Cu contained in the resulting baked material was 16 nm.

(Preparation Example 4)

<Preparation of CuNiZr/ZrO$_2$ by Impregnation Method>

The same procedure as in Preparation Example 2 was performed except that in the preparation of CuNiZn/ZrO$_2$ by the impregnation method in Preparation Example 2, 0.27 g of zinc nitrate hexahydrate (produced by Fujifilm Wako Pure Chemical Corporation) was changed to 0.18 g of zirconium nitrate dihydrate (produced by Fujifilm Wako Pure Chemical Corporation), so as to provide a CuNiZr/ZrO$_2$ baked material (powder) as the catalyst (A).

(Preparation Example 5)

<Preparation of CuNiNb/ZrO$_2$ by Impregnation Method>

The same procedure as in Preparation Example 2 was performed except that in the preparation of CuNiZn/ZrO$_2$ by the impregnation method in Preparation Example 2, 0.27 g of zinc nitrate hexahydrate (produced by Fujifilm Wako Pure Chemical Corporation) was changed to 0.20 g of niobium ammonium oxalate (produced by Sigma-Aldrich Japan LLC), so as to provide a CuNiNb/ZrO$_2$ baked material (powder) as the catalyst (A).
The average primary particle diameter of Cu contained in the resulting baked material was 16 nm.

(Preparation Example 6)

<Preparation of CuNiLa/ZrO$_2$ by Impregnation Method>

The same procedure as in Preparation Example 2 was performed except that in the preparation of CuNiZn/ZrO$_2$ by the impregnation method in Preparation Example 2, 0.27 g of zinc nitrate hexahydrate (produced by Fujifilm Wako Pure Chemical Corporation) was changed to 0.19 g of lanthanum nitrate hexahydrate (produced by Fujifilm Wako Pure Chemical Corporation), so as to provide a CuNiLa/ZrO$_2$ baked material (powder) as the catalyst (A).
The average primary particle diameter of Cu contained in the resulting baked material was 17 nm.

(Preparation Example 7)

<Preparation of CuNiCe/ZrO$_2$ by Impregnation Method>

The same procedure as in Preparation Example 2 was performed except that in the preparation of CuNiZn/ZrO$_2$ by the impregnation method in Preparation Example 2, 0.27 g of zinc nitrate hexahydrate (produced by Fujifilm Wako Pure Chemical Corporation) was changed to 0.19 g of cerous nitrate hexahydrate (produced by Fujifilm Wako Pure Chemical Corporation), so as to provide a CuNiCe/ZrO$_2$ baked material (powder) as the catalyst (A).
The average primary particle diameter of Cu contained in the resulting baked material was 17 nm.

(Preparation Example 8)

<Preparation of CuNiSm/ZrO$_2$ by Impregnation Method>

The same procedure as in Preparation Example 2 was performed except that in the preparation of CuNiZn/ZrO$_2$ by the impregnation method in Preparation Example 2, 0.27 g of zinc nitrate hexahydrate (produced by Fujifilm Wako Pure Chemical Corporation) was changed to 0.15 g of samarium chloride hexahydrate (produced by Fujifilm Wako Pure Chemical Corporation), so as to provide a CuNiSm/ZrO$_2$ baked material (powder) as the catalyst (A).

(Preparation Example 9)

<Preparation of CuNiTa/ZrO$_2$ by Impregnation Method>

The same procedure as in Preparation Example 1 was performed except that in the preparation of CuNiTi/ZrO$_2$ by the impregnation method in Preparation Example 1, 0.36 g of titanium tetraisopropoxide (produced by Fujifilm Wako Pure Chemical Corporation) was changed to 0.12 g of tantalum chloride (produced by Fujifilm Wako Pure Chemical Corporation), and the solvent was changed from 4.7 g of 2-propanol (produced by Fujifilm Wako Pure Chemical Corporation) to 4.7 g of ethanol (produced by Fujifilm Wako Pure Chemical Corporation), so as to provide a CuNiTa/ZrO$_2$ baked material (powder) as the catalyst (A).

(Preparation Example 10)

<Preparation of CuNiW/ZrO$_2$ by Impregnation Method>

The same procedure as in Preparation Example 2 was performed except that in the preparation of CuNiZn/ZrO$_2$

by the impregnation method in Preparation Example 2, 0.27 g of zinc nitrate hexahydrate (produced by Fujifilm Wako Pure Chemical Corporation) was changed to 0.08 g of tungstic acid (produced by Fujifilm Wako Pure Chemical Corporation), 6 g of ion exchanged water as a solvent was changed to 16 g thereof, and a 28% aqueous ammonia

(produced by Fujifilm Wako Pure Chemical Corporation) was added to 16 g of the ion exchanged water until the pH became 8, so as to provide a $CuNiW/ZrO_2$ baked material (powder) as the catalyst (A).

The average primary particle diameter of Cu contained in the resulting baked material was 20 nm.

(Preparation Example 11)

<Preparation of $CuNiAu/ZrO_2$ by Impregnation Method>

The same procedure as in Preparation Example 2 was performed except that in the preparation of $CuNiZn/ZrO_2$ by the impregnation method in Preparation Example 2, 0.27 g of zinc nitrate hexahydrate (produced by Fujifilm Wako Pure Chemical Corporation) was changed to 0.13 g of tetrachloroauric acid tetrahydrate (produced by Fujifilm Wako Pure Chemical Corporation), so as to provide a $CuNiAu/ZrO_2$ baked material (powder) as the catalyst (A).

(Preparation Example 12)

<Preparation of $CuNiFe/ZrO_2$ by Impregnation Method>

The same procedure as in Preparation Example 2 was performed except that in the preparation of $CuNiZn/ZrO_2$ by the impregnation method in Preparation Example 2, 0.27 g of zinc nitrate hexahydrate (produced by Fujifilm Wako Pure Chemical Corporation) was changed to 0.43 g of ferric nitrate nonahydrate (produced by Fujifilm Wako Pure Chemical Corporation), so as to provide a $CuNiFe/ZrO_2$ baked material (powder) as the catalyst (A).

(Preparation Example 13)

<Preparation of $CuNiMo/ZrO_2$ by Impregnation Method>

The same procedure as in Preparation Example 2 was performed except that in the preparation of $CuNiZn/ZrO_2$ by the impregnation method in Preparation Example 2, 0.27 g of zinc nitrate hexahydrate (produced by Fujifilm Wako Pure Chemical Corporation) was changed to 0.11 g of ammonium molybdate tetrahydrate (produced by Fujifilm Wako Pure Chemical Corporation), so as to provide a $CuNiMo/ZrO_2$ baked material (powder) as the catalyst (A).

(Preparation Example 14)

<Preparation of $CuNiRe/ZrO_2$ by Impregnation Method>

The same procedure as in Preparation Example 2 was performed except that in the preparation of $CuNiZn/ZrO_2$ by the impregnation method in Preparation Example 2, 0.27 g of zinc nitrate hexahydrate (produced by Fujifilm Wako Pure Chemical Corporation) was changed to 0.09 g of ammonium perrhenate (produced by Fujifilm Wako Pure Chemical Corporation), so as to provide a $CuNiRe/ZrO_2$ baked material (powder) as the catalyst (A).

(Comparative Preparation Example 1)

<Preparation of $Cu/ZrO_2$ by Precipitation Method>

9.5 g of cupric nitrate trihydrate (produced by Kanto Chemical Co., Inc.) was placed in a 100 mL beaker, to which 49 g of ion exchanged water was added for dissolving to prepare a cupric nitrate aqueous solution. Subsequently, 7.5 g of sodium carbonate (produced by Fujifilm Wako Pure Chemical Corporation) was placed in another 100 mL beaker, to which 42 g of ion exchanged water was added for dissolving to prepare a sodium carbonate aqueous solution. And further, 10 g of zirconium oxide (product name: RC-100 zirconium oxide, produced by Daiich Kigenso Kagaku Kyogyo Co., Ltd. (white powder, median diameter (d50): 1.5 to 4 μm)) was placed in another 2 L beaker, to which 175 g of ion exchanged water was added to prepare a slurry of zirconium oxide.

The cupric nitrate aqueous solution was added dropwise to the slurry of zirconium oxide, and simultaneously the sodium carbonate aqueous solution was added dropwise thereto, while retaining the pH to 7 (35°C) over 15 minutes. After completing the dropwise addition, a solid matter including zirconium oxide to be a carrier having a carbonate or a hydroxide of Cu attached thereto was filtrated under reduced pressure, and the resulting cake was washed with 1 L of ion exchanged water. The cake was subjected four times to an operation including re-slurrying, filtration under reduced pressure, and washing with water, then dried at 120°C for 18 hours, and further baked in air at 500°C for 3 hours, so as to provide a Cu/$ZrO_2$ baked material (powder) as a catalyst for a comparative example.

The average primary particle diameter of Cu contained in the resulting baked material was 26 nm.

(Comparative Preparation Example 2)

<Preparation of CuNiRu/$ZrO_2$ by Impregnation Method>

The same procedure as in Preparation Example 2 was performed except that in the preparation of CuNiZn/$ZrO_2$ by the impregnation method in Preparation Example 2, 0.27 g of zinc nitrate hexahydrate (produced by Fujifilm Wako Pure Chemical Corporation) was changed to 0.12 g of ruthenium chloride (produced by N.E. Chemcat Corporation), so as to provide a CuNiRu/$ZrO_2$ baked material (powder) as a catalyst for a comparative example.

(Preparation Example B)

<Preparation of CuCo/$ZrO_2$ by Precipitation Method>

11.2 g of cupric nitrate trihydrate (produced by Kanto Chemical Co., Inc.) and 2.7 g of cobalt nitrate hexahydrate (produced by Kanto Chemical Co., Inc.) were placed in a 100 mL beaker, to which 58.1 g of ion exchanged water was added for dissolving to prepare a mixed aqueous solution of cupric nitrate and cobalt nitrate having a ratio Cu/Co = 5/1 (mass ratio). Subsequently, 10.7 g of sodium carbonate (produced by Fujifilm Wako Pure Chemical Corporation) was placed in another 100 mL beaker, to which 59.1 g of ion exchanged water was added for dissolving to prepare a sodium carbonate aqueous solution. And further, 10.0 g of zirconium oxide (product name: RC-100 zirconium oxide, produced by Daiich Kigenso Kagaku Kyogyo Co., Ltd. (white powder, median diameter (d50): 1.5 to 4 μm)) was placed in another 1 L beaker, to which 175.0 g of ion exchanged water was added to prepare a slurry of zirconium oxide.

The cupric nitrate-cobalt nitrate mixed aqueous solution was added dropwise to the slurry of zirconium oxide, and simultaneously the sodium carbonate aqueous solution was added dropwise thereto, while retaining the pH to 7 (35°C) over 15 minutes. After completing the dropwise addition, a solid matter including zirconium oxide to be a carrier having a carbonate or a hydroxide of Cu and Co attached thereto was filtrated under reduced pressure, and the resulting cake was washed with 1 L of ion exchanged water. The cake was subjected three times to an operation including re-slurrying, filtration under reduced pressure, and washing with water, and then dried at 120°C for 18 hours, so as to provide a CuCo/$ZrO_2$ dried material.

(Preparation Example 15)

<Preparation of CuCoTi/$ZrO_2$ by Impregnation Method>

2.4 g of titanium tetraisopropoxide (produced by Fujifilm Wako Pure Chemical Corporation) was placed in a 100 mL recovery flask, to which 23.4 g of 2-propanol (produced by Fujifilm Wako Pure Chemical Corporation) was added for completely dissolving. 2.0 g of the CuCo/$ZrO_2$ dried material obtained in Preparation Example B was added thereto, and the mixture was concentrated until the liquid phase disappeared with a rotary evaporator, then dried at 120°C for 18 hours, and further baked in air at 500°C for 3 hours, so as to provide a CuCoTi/$ZrO_2$ baked material (powder) as the catalyst (A).

(Preparation Example C)

<Preparation of CuMo/$ZrO_2$ by Precipitation Method>

0.17 g of hexaammonium heptamolybdate tetrahydrate (produced by Fujifilm Wako Pure Chemical Corporation) was placed in a 50 mL recovery flask, to which 6.0 g of ion exchanged water was added for completely dissolving. 2.0 g of the Cu/$ZrO_2$ baked material obtained in Comparative Preparation Example 1 was added thereto, and the mixture was concentrated until the liquid phase disappeared with a rotary evaporator, and then dried at 120°C for 18 hours, so as to provide a CuMo/$ZrO_2$ dried material.

(Preparation Example 16)

<Preparation of CuMoTi/ZrO$_2$ by Impregnation Method>

0.4 g of titanium tetraisopropoxide (produced by Fujifilm Wako Pure Chemical Corporation) was placed in a 50 mL recovery flask, to which 4.7 g of 2-propanol (produced by Fujifilm Wako Pure Chemical Corporation) was added for completely dissolving. 1.9 g of the CuMo/ZrO$_2$ dried material obtained in Preparation Example C was added thereto, and then the same procedure as in Preparation Example 15 was performed, so as to provide a CuMoTi/ZrO$_2$ baked material (powder) as the catalyst (A).

(Preparation Example 17)

<Preparation of CuReTi/ZrO$_2$ by Impregnation Method>

The same procedure as in Preparation Example 16 was performed except that in the preparation of CuMoTi/ZrO$_2$ by the impregnation method in Preparation Example 16, 0.17 g of hexaammonium heptamolybdate tetrahydrate (produced by Fujifilm Wako Pure Chemical Corporation) was changed to 0.1 g of ammonium perrhenate (produced by Fujifilm Wako Pure Chemical Corporation), so as to provide a CuReTi/ZrO$_2$ baked material (powder) as the catalyst (A).

(Preparation Example D)

<Preparation of CuNi/HT by Precipitation Method>

10.1 g of cupric nitrate trihydrate (produced by Kanto Chemical Co., Inc.) and 2.9 g of nickel nitrate hexahydrate (produced by Kanto Chemical Co., Inc.) were placed in a 100 mL beaker, to which 52.0 g of ion exchanged water was added for dissolving to prepare a mixed aqueous solution of cupric nitrate and nickel nitrate having a ratio Cu/Ni = 5/1 (mass ratio). Subsequently, 9.8 g of sodium carbonate (produced by Fujifilm Wako Pure Chemical Corporation) was placed in another 100 mL beaker, to which 55.0 g of ion exchanged water was added for dissolving to prepare a sodium carbonate aqueous solution. And further, 10.0 g of hydrotalcite (product name: Kyowaad 500 PL, produced by Kyowa Chemical Industry, Co., Ltd.) was placed in another 1 L beaker, to which 175.0 g of ion exchanged water was added to prepare a slurry of hydrotalcite.
The cupric nitrate-nickel nitrate mixed aqueous solution was added dropwise to the slurry of hydrotalcite, and simultaneously the sodium carbonate aqueous solution was added dropwise thereto, while retaining the pH to 7 (35°C) over 15 minutes. After completing the dropwise addition, the same procedure as in Preparation Example B was performed, so as to provide a CuNi/HT dried material.

(Preparation Example 18)

<Preparation of CuNiTi/HT by Impregnation Method>

0.9 g of titanium tetraisopropoxide (produced by Fujifilm Wako Pure Chemical Corporation) was placed in a 100 mL recovery flask, to which 11.7 g of 2-propanol (produced by Fujifilm Wako Pure Chemical Corporation) was added for completely dissolving. 5.0 g of the CuNi/HT dried material obtained in Preparation Example D was added thereto, and then the same procedure as in Preparation Example 15 was performed, so as to provide a CuNiTi/HT baked material (powder) as the catalyst (A).

(Preparation Example E)

<Preparation of CuNi/Al$_2$O$_3$ by Precipitation Method>

10.4 g of cupric nitrate trihydrate (produced by Kanto Chemical Co., Inc.) and 2.7 g of nickel nitrate hexahydrate (produced by Kanto Chemical Co., Inc.) were placed in a 100 mL beaker, to which 53.9 g of ion exchanged water was added for dissolving to prepare a mixed aqueous solution of cupric nitrate and nickel nitrate having a ratio Cu/Ni = 5/1 (mass ratio). Subsequently, 10.0 g of sodium carbonate (produced by Fujifilm Wako Pure Chemical Corporation) was placed in another 100 mL beaker, to which 55.6 g of ion exchanged water was added for dissolving to prepare a sodium carbonate aqueous solution. And further, 10.0 g of aluminum oxide (product name: SA6278, produced by Saint-Gobain S.A.) was placed in another 1 L beaker, to which 175.0 g of ion exchanged water was added to prepare a slurry of aluminum oxide.

The cupric nitrate-nickel nitrate mixed aqueous solution was added dropwise to the slurry of aluminum oxide, and simultaneously the sodium carbonate aqueous solution was added dropwise thereto, while retaining the pH to 7 (35°C) over 15 minutes. After completing the dropwise addition, the same procedure as in Preparation Example B was performed, so as to provide a CuNi/Al$_2$O$_3$ dried material.

(Preparation Example 19)

<Preparation of CuNiTi/Al$_2$O$_3$ by Impregnation Method>

2.4 g of titanium tetraisopropoxide (produced by Fujifilm Wako Pure Chemical Corporation) was placed in a 100 mL recovery flask, to which 31.7 g of 2-propanol (produced by Fujifilm Wako Pure Chemical Corporation) was added for completely dissolving. 13.4 g of the CuNi/Al$_2$O$_3$ dried material obtained in Preparation Example E was added thereto, and then the same procedure as in Preparation Example 15 was performed, so as to provide a CuNiTi/Al$_2$O$_3$ baked material (powder) as the catalyst (A).

(Comparative Preparation Example 3)

<Preparation of Cu/HT by Precipitation Method>

4.8 g of cupric nitrate trihydrate (produced by Kanto Chemical Co., Inc.) was placed in a 50 mL beaker, to which 25.0 g of ion exchanged water was added for dissolving to prepare a cupric nitrate aqueous solution. Subsequently, 3.8 g of sodium carbonate (produced by Fujifilm Wako Pure Chemical Corporation) was placed in another 50 mL beaker, to which 21.0 g of ion exchanged water was added for dissolving to prepare a sodium carbonate aqueous solution. And further, 5.0 g of hydrotalcite (product name: Kyowaad 500 PL, produced by Kyowa Chemical Industry, Co., Ltd.) was placed in another 1 L beaker, to which 87.5 g of ion exchanged water was added to prepare a slurry of hydrotalcite.
The cupric nitrate aqueous solution was added dropwise to the slurry of hydrotalcite, and simultaneously the sodium carbonate aqueous solution was added dropwise thereto, while retaining the pH to 7 (35°C) over 15 minutes. After completing the dropwise addition, the same procedure as in Comparative Preparation Example 1 was performed, so as to provide a Cu/HT baked material (powder) as a catalyst for a comparative example.

(Comparative Preparation Example 4)

<Preparation of Cu/Al$_2$O$_3$ by Precipitation Method>

9.5 g of cupric nitrate trihydrate (produced by Kanto Chemical Co., Inc.) was placed in a 100 mL beaker, to which 49.0 g of ion exchanged water was added for dissolving to prepare a cupric nitrate aqueous solution. Subsequently, 7.5 g of sodium carbonate (produced by Fujifilm Wako Pure Chemical Corporation) was placed in another 50 mL beaker, to which 42.0 g of ion exchanged water was added for dissolving to prepare a sodium carbonate aqueous solution. And further, 10.0 g of crushed aluminum oxide (product name: SA6278, produced by Saint-Gobain S.A.) was placed in another 1 L beaker, to which 175.0 g of ion exchanged water was added to prepare a slurry of aluminum oxide.
The cupric nitrate aqueous solution was added dropwise to the slurry of aluminum oxide, and simultaneously the sodium carbonate aqueous solution was added dropwise thereto, while retaining the pH to 7 (35°C) over 15 minutes. After completing the dropwise addition, the same procedure as in Comparative Preparation Example 1 was performed, so as to provide a Cu/Al$_2$O$_3$ baked material (powder) as a catalyst for a comparative example.

(Preparation Example F)

<Preparation of CuNi/ZrO$_2$ by Precipitation Method>

14.5 g of cupric nitrate trihydrate (produced by Kanto Chemical Co., Inc.) and 3.9 g of nickel nitrate hexahydrate (produced by Kanto Chemical Co., Inc.) were placed in a 100 mL beaker, to which 77.1 g of ion exchanged water was added for dissolving to prepare a mixed aqueous solution of cupric nitrate and nickel nitrate having a ratio Cu/Ni = 5/1 (mass ratio). Subsequently, 14.3 g of sodium carbonate (produced by Fujifilm Wako Pure Chemical Corporation) was placed in another 100 mL beaker, to which 79.4 g of ion exchanged water was added for dissolving to prepare a sodium carbonate aqueous solution. And further, 5.0 g of zirconium oxide (product name: RC-100 zirconium oxide, produced by Daiich Kigenso Kagaku Kyogyo Co., Ltd. (white powder, median diameter (d50): 1.5 to 4 $\mu$m)) was placed in another 1 L beaker, to which 87.5 g of ion exchanged water was added to prepare a slurry of zirconium oxide.
The cupric nitrate-nickel nitrate mixed aqueous solution was added dropwise to the slurry of zirconium oxide, and simultaneously the sodium carbonate aqueous solution was added dropwise thereto, while retaining the pH to 7 (35°C) over 24 minutes. After completing the dropwise addition, the same procedure as in Preparation Example B was performed, so as to provide a CuNi/ZrO$_2$ dried material.

(Preparation Example 20)

<Preparation of CuNiTi/ZrO$_2$ by Impregnation Method>

0.6 g of titanium tetraisopropoxide (produced by Fujifilm Wako Pure Chemical Corporation) was placed in a 100 mL recovery flask, to which 7.7 g of 2-propanol (produced by Fujifilm Wako Pure Chemical Corporation) was added for completely dissolving. 12.1 g of the CuNi/ZrO$_2$ dried material obtained in Preparation Example F was added thereto, and then the same procedure as in Preparation Example 15 was performed, so as to provide a CuNiTi/ZrO$_2$ baked material (powder) as the catalyst (A).
The average primary particle diameter of Cu contained in the resulting baked material was 24 nm.

(Preparation Example G)

<Preparation of CuNi/ZrO$_2$ by Precipitation Method>

12.9 g of cupric nitrate trihydrate (produced by Kanto Chemical Co., Inc.) and 16.8 g of nickel nitrate hexahydrate (produced by Kanto Chemical Co., Inc.) were placed in a 100 mL beaker, to which 66.7 g of ion exchanged water was added for dissolving to prepare a mixed aqueous solution of cupric nitrate and nickel nitrate having a ratio Cu/Ni = 1/1 (mass ratio). Subsequently, 21.2 g of sodium carbonate (produced by Fujifilm Wako Pure Chemical Corporation) was placed in another 200 mL beaker, to which 117.7 g of ion exchanged water was added for dissolving to prepare a sodium carbonate aqueous solution. And further, 10.0 g of zirconium oxide (product name: RC-100 zirconium oxide, produced by Daiich Kigenso Kagaku Kyogyo Co., Ltd. (white powder, median diameter (d50): 1.5 to 4 $\mu$m)) was placed in another 1 L beaker, to which 175.0 g of ion exchanged water was added to prepare a slurry of zirconium oxide.
The cupric nitrate-nickel nitrate mixed aqueous solution was added dropwise to the slurry of zirconium oxide, and simultaneously the sodium carbonate aqueous solution was added dropwise thereto, while retaining the pH to 7 (35°C) over 23 minutes. After completing the dropwise addition, the same procedure as in Preparation Example B was performed, so as to provide a CuNi/ZrO$_2$ dried material.

(Preparation Example 21)

<Preparation of CuNiTi/ZrO$_2$ by Impregnation Method>

1.0 g of titanium tetraisopropoxide (produced by Fujifilm Wako Pure Chemical Corporation) was placed in a 100 mL recovery flask, to which 13.1 g of 2-propanol (produced by Fujifilm Wako Pure Chemical Corporation) was added for completely dissolving. 19.2 g of the CuNi/ZrO$_2$ dried material obtained in Preparation Example G was added thereto, and then the same procedure as in Preparation Example 15 was performed, so as to provide a CuNiTi/ZrO$_2$ baked material (powder) as the catalyst (A).
The average primary particle diameter of Cu contained in the resulting baked material was 8 nm.

(Preparation Example 22)

<Preparation of CuNiTi/ZrO$_2$ by Impregnation Method>

The same procedure as in Preparation Example 1 was performed except that in the preparation of CuNiTi/ZrO$_2$ by the impregnation method in Preparation Example 1, 0.36 g of titanium tetraisopropoxide (produced by Fujifilm Wako Pure Chemical Corporation) was changed to 0.3 g thereof, 4.7 g of 2-propanol (produced by Fujifilm Wako Pure Chemical Corporation) was changed to 11.7 g thereof, and 2.0 g CuNi/ZrO$_2$ baked material was changed to 5.0 g thereof, so as to provide a CuNiTi/ZrO$_2$ baked material (powder) as the catalyst (A).

(Preparation Example 23)

<Preparation of CuNiTi/ZrO$_2$ by Impregnation Method>

The same procedure as in Preparation Example 22 was performed except that in the preparation of CuNiTi/ZrO$_2$

by the impregnation method in Preparation Example 22, 0.3 g of titanium tetraisopropoxide (produced by Fujifilm Wako Pure Chemical Corporation) was changed to 1.2 g thereof, so as to provide a CuNiTi/ZrO$_2$ baked material (powder) as the catalyst (A).

(Preparation Example H)

<Preparation of CuNi/ZrO$_2$ by Precipitation Method>

1.3 g of cupric nitrate trihydrate (produced by Kanto Chemical Co., Inc.) and 1.6 g of nickel nitrate hexahydrate (produced by Kanto Chemical Co., Inc.) were placed in a 50 mL beaker, to which 6.5 g of ion exchanged water was added for dissolving to prepare a mixed aqueous solution of cupric nitrate and nickel nitrate having a ratio Cu/Ni = 1/1 (mass ratio). Subsequently, 2.1 g of sodium carbonate (produced by Fujifilm Wako Pure Chemical Corporation) was placed in another 50 mL beaker, to which 11.5 g of ion exchanged water was added for dissolving to prepare a sodium carbonate aqueous solution. And further, 10.0 g of zirconium oxide (product name: RC-100 zirconium oxide, produced by Daiich Kigenso Kagaku Kyogyo Co., Ltd. (white powder, median diameter (d50): 1.5 to 4 μm)) was placed in another 1 L beaker, to which 175.0 g of ion exchanged water was added to prepare a slurry of zirconium oxide.
The cupric nitrate-nickel nitrate mixed aqueous solution was added dropwise to the slurry of zirconium oxide, and simultaneously the sodium carbonate aqueous solution was added dropwise thereto, while retaining the pH to 7 (35°C) over 2 minutes. After completing the dropwise addition, the same procedure as in Preparation Example B was performed, so as to provide a CuNi/ZrO$_2$ dried material.

(Preparation Example 24)

<Preparation of CuNiTi/ZrO$_2$ by Impregnation Method>

0.6 g of titanium tetraisopropoxide (produced by Fujifilm Wako Pure Chemical Corporation) was placed in a 50 mL recovery flask, to which 8.5 g of 2-propanol (produced by Fujifilm Wako Pure Chemical Corporation) was added for completely dissolving. 8.5 g of the CuNi/ZrO$_2$ dried material obtained above was added thereto, and then the same procedure as in Preparation Example 15 was performed, so as to provide a CuNiTi/ZrO$_2$ baked material (powder) as the catalyst (A).
The average primary particle diameter of Cu contained in the resulting baked material was 5 nm.

(Comparative Preparation Example 5)

<Preparation of Cu/ZrO$_2$ by Impregnation Method>

15.2 g of cupric nitrate trihydrate (produced by Kanto Chemical Co., Inc.) was placed in a 100 mL recovery flask, to which 30.0 g of ion exchanged water was added for completely dissolving. 10.0 g of zirconium oxide (product name: RC-100 zirconium oxide, produced by Daiich Kigenso Kagaku Kyogyo Co., Ltd. (white powder, median diameter (d50): 1.5 to 4 μm)) was added thereto, and the mixture was concentrated until the liquid phase disappeared with a rotary evaporator, then dried at 120°C for 18 hours, and baked in air at 500°C for 3 hours, so as to provide a Cu/ZrO$_2$ baked material (powder) as a catalyst for a comparative example.

(Comparative Preparation Example 6)

<Preparation of Cu/ZrO$_2$ by Precipitation Method>

1.2 g of cupric nitrate trihydrate (produced by Kanto Chemical Co., Inc.) was placed in a 50 mL beaker, to which 6.0 g of ion exchanged water was added for dissolving to prepare a cupric nitrate aqueous solution. Subsequently, 1.0 g of sodium carbonate (produced by Fujifilm Wako Pure Chemical Corporation) was placed in another 50 mL beaker, to which 5.4 g of ion exchanged water was added for dissolving to prepare a sodium carbonate aqueous solution. And further, 10 g of zirconium oxide (product name: RC-100 zirconium oxide, produced by Daiich Kigenso Kagaku Kyogyo Co., Ltd. (white powder, median diameter (d50): 1.5 to 4 μm)) was placed in another 1 L beaker, to which 175 g of ion exchanged water was added to prepare a slurry of zirconium oxide.
The cupric nitrate aqueous solution was added dropwise to the slurry of zirconium oxide, and simultaneously the sodium carbonate aqueous solution was added dropwise thereto, while retaining the pH to 7 (35°C) over 2 minutes. After completing the dropwise addition, the same procedure as in Comparative Preparation Example 1 was performed, so as to provide a Cu/ZrO$_2$ baked material (powder) as a catalyst for a comparative example.

The average primary particle diameter of Cu contained in the resulting baked material was 5 nm.

[Examples 1 to 14 and Comparative Examples 1 to 4]

Investigation into Third Component

(Example 1)

In a 1 L five-neck glass flask equipped with a stirrer, a thermometer, a nitrogen blowing tube, a sampling tube, and a condenser and a dephlegmator for isolating by-produced water in reaction, 600.0 g (3.22 mol) of 1-dodecanol (C12) (product name: Kalcol 2098, produced by Kao Corporation) as the raw material alcohol, 1.13 g (0.3% by mol based on the raw material alcohol) of a 48% potassium hydroxide aqueous solution (produced by Kanto Chemical Co., Inc.) as the base catalyst (B), and 0.6 g (0.1% by mass based on the raw material alcohol) of the $CuNiTi/ZrO_2$ baked material prepared in Preparation Example 1 as the catalyst (A) were charged, the system was heated while bubbling nitrogen gas into the system at a flow rate of 6 L/hr. After the time when the temperature in the system reached 240°C, the flow rate of nitrogen gas was changed to 0.13 L/hr, and the reaction was performed for 3 hours. The production condition and the result are shown in Table 1.

(Examples 2 to 14 and Comparative Examples 1 to 4)

The reaction was performed in the same manner as in Example 1 except that the kind of the catalyst (A) and the reaction time were changed as shown in Table 1. The results are shown in Table 1.

[Examples 15 to 17 and Comparative Examples 1 to 3]

Investigation into Second Component

The reaction was performed in the same manner as in Example 1 except that the kind of the catalyst (A) and the reaction time were changed as shown in Table 2. The results are shown in Table 2.

[Examples 18 to 20 and Comparative Examples 5 to 7]

Investigation into Raw Material Alcohol and Carrier

The reaction was performed in the same manner as in Example 1 except that the kind of the raw material alcohol, the kind of the catalyst (A), and the reaction time were changed as shown in Table 3. The results are shown in Table 3.

[Examples 21 to 25 and Comparative Examples 1, 8, and 9]

Investigation into Supported Amount

The reaction was performed in the same manner as in Example 1 except that the kind of the catalyst (A) and the reaction time were changed as shown in Table 4. The results are shown in Table 4.

# Table 1: Investigation into Third Component

| | | Example 1 | Example 2 | Example 3 | Example 4 | Example 5 | Example 6 | Example 7 | Example 8 | Example 9 |
|---|---|---|---|---|---|---|---|---|---|---|
| Raw material alcohol | | 1-dodecanol | 1-dodecanol | 1-dodecanol | 1-dodecanol | 1-dodecanol | 1-dodecanol | 1-dodecanol | 1-dodecanol | 1-dodecanol |
| Base catalyst (B) | Kind | KOH | KOH | KOH | KOH | KOH | KOH | KOH | KOH | KOH |
| | Added amount (% by mol based on raw material alcohol) | 0.3 | 0.3 | 0.3 | 0.3 | 0.3 | 0.3 | 0.3 | 0.3 | 0.3 |
| Catalyst (A) | Kind | Preparation Example 1 | Preparation Example 2 | Preparation Example 3 | Preparation Example 4 | Preparation Example 5 | Preparation Example 6 | Preparation Example 7 | Preparation Example 8 | Preparation Example 9 |
| | Added amount (% by mass based on raw material alcohol) | 0.1 | 0.1 | 0.1 | 0.1 | 0.1 | 0.1 | 0.1 | 0.1 | 0.1 |
| | First component — Kind | Cu | Cu | Cu | Cu | Cu | Cu | Cu | Cu | Cu |
| | First component — Content (% by mass) | 17.0 | 17.0 | 17.0 | 17.0 | 17.0 | 17.0 | 17.0 | 17.0 | 17.0 |
| | Second component — Kind | Ni | Ni | Ni | Ni | Ni | Ni | Ni | Ni | Ni |
| | Second component — Content (% by mass) | 3.0 | 3.1 | 3.0 | 3.1 | 2.9 | 3.0 | 3.1 | 3.0 | 3.0 |
| | Third component — Kind | Ti | Zn | Y | Zr | Nb | La | Ce | Sm | Ta |
| | Third component — Content (% by mass) | 2.9 | 2.9 | 2.8 | 2.9 | 2.8 | 2.8 | 2.9 | 2.8 | 2.8 |
| | Carrier — Kind | $ZrO_2$ | $ZrO_2$ | $ZrO_2$ | $ZrO_2$ | $ZrO_2$ | $ZrO_2$ | $ZrO_2$ | $ZrO_2$ | $ZrO_2$ |
| | Second component/ first component (mass ratio) | 0.18 | 0.18 | 0.18 | 0.18 | 0.17 | 0.18 | 0.18 | 0.18 | 0.18 |
| | Third component/ first component (mass ratio) | 0.17 | 0.17 | 0.16 | 0.17 | 0.16 | 0.16 | 0.17 | 0.16 | 0.16 |
| Reaction condition | Reaction temperature (°C) | 240 | 240 | 240 | 240 | 240 | 240 | 240 | 240 | 240 |
| | Reaction time (h) | 3 | 8 | 7 | 8 | 3 | 6 | 6 | 5 | 4 |
| Evaluation | Conversion of raw material alcohol (%) | 41 | 42 | 43 | 41 | 42 | 39 | 42 | 42 | 40 |
| | By-production rate of dimer ester (%) | 0.5 | 0.8 | 0.5 | 0.6 | 0.6 | 0.5 | 0.5 | 0.5 | 0.4 |

EP 4 079 719 A1

23

Table 1 (continued): Investigation into Third Component

| | | | Example 10 | Example 11 | Comparative Example 1 | Example 12 | Example 13 | Comparative Example 2 | Example 14 | Comparative Example 3 | Comparative Example 4 |
|---|---|---|---|---|---|---|---|---|---|---|---|
| | Raw material alcohol | | 1-dodecanol | 1-dodecanol | 1-dodecanol | 1-dodecanol | 1-dodecanol | 1-dodecanol | 1-dodecanol | 1-dodecanol | 1-dodecanol |
| Base catalyst (B) | Kind | | KOH | KOH | KOH | KOH | KOH | KOH | KOH | KOH | KOH |
| | Added amount (% by mol based on raw material alcohol) | | 0.3 | 0.3 | 0.3 | 0.3 | 0.3 | 0.3 | 0.3 | 0.3 | 0.3 |
| Catalyst (A) | Kind | | Preparation Example 10 | Preparation Example 11 | Comparative Preparation Example 1 | Preparation Example 12 | Preparation Example 13 | Comparative Preparation Example 1 | Preparation Example 14 | Comparative Preparation Example 1 | Comparative Preparation Example 2 |
| | Added amount (% by mass based on raw material alcohol) | | 0.1 | 0.1 | 0.1 | 0.1 | 0.1 | 0.1 | 0.1 | 0.1 | 0.1 |
| | First component | Kind | Cu | Cu | Cu | Cu | Cu | Cu | Cu | Cu | Cu |
| | | Content (% by mass) | 17.0 | 17.0 | 18.0 | 17.0 | 17.0 | 18.0 | 17.0 | 18.0 | 17.0 |
| | Second component | Kind | Ni | Ni | - | Ni | Ni | - | Ni | - | Ni |
| | | Content (% by mass) | 3.1 | 3.1 | - | 3.1 | 3.1 | - | 3.1 | - | 3.1 |
| | Third component | Kind | W | Au | - | Fe | Mo | - | Re | - | Ru |
| | | Content (% by mass) | 2.9 | 2.9 | - | 2.9 | 2.9 | - | 2.9 | - | 2.9 |
| | Carrier | Kind | $ZrO_2$ | $ZrO_2$ | $ZrO_2$ | $ZrO_2$ | $ZrO_2$ | $ZrO_2$ | $ZrO_2$ | $ZrO_2$ | $ZrO_2$ |
| | Second component/ first component (mass ratio) | | 0.18 | 0.18 | - | 0.18 | 0.18 | - | 0.18 | - | 0.18 |
| | Third component/ first component (mass ratio) | | 0.17 | 0.17 | - | 0.17 | 0.17 | - | 0.17 | - | 0.17 |
| Reaction condition | Reaction temperature (°C) | | 240 | 240 | 240 | 240 | 240 | 240 | 240 | 240 | 240 |
| | Reaction time (h) | | 2 | 7 | 3 | 8 | 3 | 2 | 7 | 1 | 6 |
| Evaluation | Conversion of raw material alcohol (%) | | 37 | 41 | 41 | 31 | 32 | 33 | 20 | 23 | 21 |
| | By-production rate of dimer ester (%) | | 0.6 | 0.7 | 1.0 | 0.3 | 0.3 | 0.6 | 0.1 | 0.3 | 0.3 |

24

Table 2: Investigation into Second Component

| | | | Example 15 | Comparative Example 2 | Example 16 | Comparative Example 1 | Example 17 | Comparative Example 3 |
|---|---|---|---|---|---|---|---|---|
| Raw material alcohol | | | 1-dodecanol | 1-dodecanol | 1-dodecanol | 1-dodecanol | 1-dodecanol | 1-dodecanol |
| Base catalyst (B) | Kind | | KOH | KOH | KOH | KOH | KOH | KOH |
| | Added amount (% by mol based on raw material alcohol) | | 0.3 | 0.3 | 0.3 | 0.3 | 0.3 | 0.3 |
| Catalyst (A) | Kind | | Preparation Example 15 | Comparative Preparation Example1 | Preparation Example 16 | Comparative Preparation Example1 | Preparation Example 17 | Comparative Preparation Example1 |
| | Added amount (% by mol based on raw material alcohol) | | 0.1 | 0.1 | 0.1 | 0.1 | 0.1 | 0.1 |
| | First component | Kind | Cu | Cu | Cu | Cu | Cu | Cu |
| | | Content (% by mass) | 20.0 | 18.0 | 18.0 | 18.0 | 18.0 | 18.0 |
| | Second component | Kind | Co | - | Mo | - | Re | - |
| | | Content (% by mass) | 3.3 | - | 4.2 | - | 3.5 | - |
| | Third component | Kind | Ti | - | Ti | - | Ti | - |
| | | Content (% by mass) | 3.2 | - | 2.9 | - | 3.1 | - |
| | Carrier | Kind | $ZrO_2$ | $ZrO_2$ | $ZrO_2$ | $ZrO_2$ | $ZrO_2$ | $ZrO_2$ |
| | Second component/ first component (mass ratio) | | 0.17 | - | 0.23 | - | 0.19 | - |
| | Third component/ first component (mass ratio) | | 0.16 | - | 0.16 | - | 0.17 | - |
| Reaction condition | Reaction temperature (°C) | | 240 | 240 | 240 | 240 | 240 | 240 |
| | Reaction time (h) | | 7 | 2 | 3 | 3 | 8 | 1 |

EP 4 079 719 A1

| | | Example 15 | Comparative Example 2 | Example 16 | Comparative Example 1 | Example 17 | Comparative Example 3 |
|---|---|---|---|---|---|---|---|
| Evaluation | Conversion of raw material alcohol (%) | 31 | 33 | 44 | 41 | 19 | 23 |
| | By-production rate of dimer ester (%) | 0.2 | 0.6 | 0.6 | 1.0 | 0.1 | 0.3 |

Table 3: Investigation into Raw Material Alcohol and Carrier

| | | | Example 18 | Comparative Example 5 | Example 19 | Comparative Example 6 | Example 20 | Comparative Example 7 |
|---|---|---|---|---|---|---|---|---|
| Raw material alcohol | | | 1-dodecanol | 1-dodecanol | 1-decanol | 1-decanol | 1-octadecanol | 1-octadecanol |
| Base catalyst (B) | Kind | | KOH | KOH | KOH | KOH | KOH | KOH |
| | Added amount (% by mol based on raw material alcohol) | | 0.3 | 0.3 | 0.3 | 0.3 | 0.3 | 0.3 |
| Catalyst (A) | Kind | | Preparation Example 18 | Comparative Preparation Example3 | Preparation Example 19 | Comparative Preparation Example4 | Preparation Example1 | Comparative Preparation Example1 |
| | Added amount (% by mol based on raw material alcohol) | | 0.1 | 0.1 | 0.1 | 0.1 | 0.1 | 0.1 |
| | First component | Kind | Cu | Cu | Cu | Cu | Cu | Cu |
| | | Content (% by mass) | 25.00 | 28.00 | 20.00 | 17.00 | 17.00 | 18.00 |
| | Second component | Kind | Ni | - | Ni | - | Ni | - |
| | | Content (% by mass) | 4.8 | - | 3.4 | - | 3.0 | - |
| | Third component | Kind | Ti | - | Ti | - | Ti | - |
| | | Content (% by mass) | 3.0 | - | 3.1 | - | 2.9 | - |
| | Carrier | Kind | HT | HT | $Al_2O_3$ | $Al_2O_3$ | $ZrO_2$ | $ZrO_2$ |
| | Second component/ first component (mass ratio) | | 0.19 | - | 0.17 | - | 0.18 | - |
| | Third component/ first component (mass ratio) | | 0.12 | - | 0.16 | - | 0.17 | - |
| Reaction condition | Reaction temperature (°C) | | 240 | 240 | 240 | 240 | 240 | 240 |
| | Reaction time (h) | | 2 | 2 | 8 | 4 | 7 | 2 |

(continued)

| Evaluation | | Example 18 | Comparative Example 5 | Example 19 | Comparative Example 6 | Example 20 | Comparative Example 7 |
|---|---|---|---|---|---|---|---|
| | Conversion of raw material alcohol (%) | 42 | 39 | 38 | 41 | 21 | 21 |
| | By-production rate of dimer ester (%) | 2.3 | 3.7 | 0.3 | 1.1 | 1.8 | 2.5 |

Table 4: Investigation into Supported Amount

| | | Example 21 | Comparative Example 8 | Example 22 | Example 23 | Example 24 | Comparative Example 1 | Example 25 | Comparative Example 9 |
|---|---|---|---|---|---|---|---|---|---|
| Raw material alcohol | | 1-dodecanol | 1-dodecanol | 1-dodecanol | 1-dodecanol | 1-dodecanol | 1-dodecanol | 1-dodecanol | 1-dodecanol |
| Base catalyst (B) | Kind | KOH | KOH | KOH | KOH | KOH | KOH | KOH | KOH |
| | Added amount (% by mol based on raw material alcohol) | 0.3 | 0.3 | 0.3 | 0.3 | 0.3 | 0.3 | 0.3 | 0.3 |

EP 4 079 719 A1

(continued)

| | | | Example 21 | Comparative Example 8 | Example 22 | Example 23 | Example 24 | Comparative Example 1 | Example 25 | Comparative Example 9 |
|---|---|---|---|---|---|---|---|---|---|---|
| Catalyst (A) | Kind | | Preparation Example20 | Comparative Preparation Example5 | Preparation Example21 | Preparation Example22 | Preparation Example23 | Comparative Preparation Example1 | Preparation Example24 | Comparative Preparation Example6 |
| | Added amount (% by mol based on raw material alcohol) | | 0.1 | 0.1 | 0.1 | 0.1 | 0.1 | 0.1 | 0.1 | 0.1 |
| | First component | Kind | Cu | Cu | Cu | Cu | Cu | Cu | Cu | Cu |
| | | Content (% by mass) | 36.0 | 40.0 | 19.0 | 17.0 | 16.0 | 18.0 | 3.0 | 2.8 |
| | Second component | Kind | Ni | - | Ni | Ni | Ni | - | Ni | - |
| | | Content (% by mass) | 6.6 | - | 17.0 | 3.0 | 3.0 | - | 2.0 | - |
| | Third component | Kind | Ti | - | Ti | Ti | Ti | - | Ti | - |
| | | Content (% by mass) | 1.0 | - | 1.0 | 1.0 | 4.0 | - | 1.2 | - |
| | Carrier | Kind | $ZrO_2$ | $ZrO_2$ | $ZrO_2$ | $ZrO_2$ | $ZrO_2$ | $ZrO_2$ | $ZrO_2$ | $ZrO_2$ |
| | Second component/ first component (mass ratio) | | 0.18 | - | 0.89 | 0.18 | 0.19 | - | 0.67 | - |
| | Third component/ first component (mass ratio) | | 0.03 | - | 0.05 | 0.06 | 0.25 | - | 0.40 | - |
| Reaction condition | Reaction temperature (°C) | | 240 | 240 | 240 | 240 | 240 | 240 | 240 | 240 |
| | Reaction time (h) | | 7 | 5 | 6 | 3 | 7 | 3 | 6 | 6 |

(continued)

| | | Example 21 | Comparative Example 8 | Example 22 | Example 23 | Example 24 | Comparative Example 1 | Example 25 | Comparative Example 9 |
|---|---|---|---|---|---|---|---|---|---|
| Evaluation | Conversion of raw material alcohol (%) | 52 | 52 | 43 | 39 | 40 | 41 | 40 | 41 |
| | By-production rate of dimer ester (%) | 0.8 | 1.1 | 0.6 | 0.6 | 0.4 | 1.0 | 0.3 | 0.5 |

(Summary of Results 1: Investigation into Third Component)

Examples 1 to 14 were performed for the investigation with the catalysts (A) having various kinds of the third component defined in the present invention, and compared with Comparative Examples 1 to 3 using the catalysts not containing the second component and the third component defined in the present invention, and with Comparative Example 4 containing Ru, which was not the third component defined in the present invention. The results are shown in Table 1.

The following matters were understood from the results of Table 1.

It was understood that Examples 1 to 11 using Ti, Zn, Y, Zr, Nb, La, Ce, Sm, Ta, W, and Au as the third component had a low by-production rate of the dimer ester (C24 ester) and thus was excellent in effect of suppressing the formation of the by-produced ester, as compared to Comparative Example 1 having the equivalent conversion of the raw material alcohol.

It was understood that Examples 12 and 13 using Fe and Mo as the third component had a low by-production rate of the dimer ester (C24 ester) and thus was excellent in effect of suppressing the formation of the by-produced ester, as compared to Comparative Example 2 having the equivalent conversion of the raw material alcohol.

It was understood that Example 14 using Re as the third component had a low by-production rate of the dimer ester (C24 ester) and thus was excellent in effect of suppressing the formation of the by-produced ester, as compared to Comparative Examples 3 and 4 having the equivalent conversion of the raw material alcohol.

(Summary of Results 2: Investigation into Second Component)

Examples 15 to 17 were performed for the investigation with the catalysts (A) having various kinds of the second component defined in the present invention, and compared with Comparative Examples 2, 1, and 3 using the catalysts not containing the second component and the third component defined in the present invention. The results are shown in Table 2. The following matters were understood from the results of Table 2.

It was understood that Example 15 using Co as the second component had a low by-production rate of the dimer ester (C24 ester) and thus was excellent in effect of suppressing the formation of the by-produced ester, as compared to Comparative Example 2 having the equivalent conversion of the raw material alcohol.

It was understood that Example 16 using Mo as the second component had a low by-production rate of the dimer ester (C24 ester) and thus was excellent in effect of suppressing the formation of the by-produced ester, as compared to Comparative Example 1 having the equivalent conversion of the raw material alcohol.

It was understood that Example 17 using Re as the second component had a low by-production rate of the dimer ester (C24 ester) and thus was excellent in effect of suppressing the formation of the by-produced ester, as compared to Comparative Example 3 having the equivalent conversion of the raw material alcohol.

(Summary of Results 3: Investigation into Raw Material Alcohol and Carrier)

Examples 18 to 20 were performed for the investigation with the catalysts (A) having various kinds of the carrier with the various raw material alcohols, in which the raw material alcohols and the carriers corresponding to Examples 18 to 20 were used, and compared with Comparative Examples 5 to 7 using the catalysts not containing the second component and the third component defined in the present invention. The results are shown in Table 3. The following matters were understood from the results of Table 3.

It was understood that Example 18 using 1-dodecanol (C12) as the raw material alcohol and HT as the carrier had a low by-production rate of the dimer ester (C24 ester) and thus was excellent in effect of suppressing the formation of the by-produced ester, as compared to Comparative Example 5 having the equivalent conversion of the raw material alcohol.

It was understood that Example 19 using 1-decanol (C10) as the raw material alcohol and $Al_2O_3$ as the carrier had a low by-production rate of the dimer ester (C20 ester) and thus was excellent in effect of suppressing the formation of the by-produced ester, as compared to Comparative Example 6 having the equivalent conversion of the raw material alcohol.

It was understood that Example 20 using 1-octadecanol (C18) as the raw material alcohol and $ZrO_2$ as the carrier had a low by-production rate of the dimer ester (C36 ester) and thus was excellent in effect of suppressing the formation of the by-produced ester, as compared to Comparative Example 7 having the equivalent conversion of the raw material alcohol.

(Summary of Results 4: Investigation into Supported Amount)

Examples 21 to 25 were performed for the investigation with the catalysts (A) having various contents (supported amounts) of the first to third components, and compared with Comparative Examples 8, 1, and 9 using the catalysts not containing the second component and the third component defined in the present invention. The results are shown in Table 4. The following matters were understood from the results of Table 4.

It was understood that Example 21 using the catalyst (A) having a mass ratio of the second component to the first component of 0.18 and a mass ratio of the third component to the first component of 0.03 had a low by-production rate of the dimer ester (C24 ester) and thus was excellent in effect of suppressing the formation of the by-produced ester, as compared to Comparative Example 8 having the equivalent conversion of the raw material alcohol.

It was understood that Examples 22 to 24 using the catalysts (A) having a mass ratio of the second component to the first component of 0.18 to 0.89 and a mass ratio of the third component to the first component of 0.05 to 0.25 had a low by-production rate of the dimer ester (C24 ester) and thus was excellent in effect of suppressing the formation of the by-produced ester, as compared to Comparative Example 1 having the equivalent conversion of the raw material alcohol.

It was understood that Example 25 using the catalyst (A) having a mass ratio of the second component to the first component of 0.67 and a mass ratio of the third component to the first component of 0.40 had a low by-production rate of the dimer ester (C24 ester) and thus was excellent in effect of suppressing the formation of the by-produced ester, as compared to Comparative Example 9 having the equivalent conversion of the raw material alcohol.

**Claims**

1.  A method for producing a Guerbet alcohol, comprising reacting a raw material alcohol having 8 or more and 22 or less carbon atoms, in the presence of a catalyst (A) containing a first component, a second component, and a third component below:

    first component: copper,
    second component: one kind selected from the group consisting of cobalt, nickel, molybdenum, and rhenium, and
    third component: at least one kind selected from the group consisting of elements that are elements belonging to Groups 3 to 10 and 12 of the fourth period of the periodic table and elements belonging to Groups 3 to 7, 11, and 12 of the fifth and sixth periods of the periodic table, and are different from the element selected as the second component.

2.  The method for producing a Guerbet alcohol according to claim 1, wherein the third component of the catalyst (A) is at least one kind selected from the group consisting of titanium, iron, zinc, yttrium, zirconium, niobium, molybdenum, lanthanum, cerium, samarium, tantalum, tungsten, rhenium, and gold.

3.  The method for producing a Guerbet alcohol according to claim 1 or 2, wherein the second component of the catalyst (A) is one kind selected from the group consisting of cobalt, nickel, and rhenium.

4.  The method for producing a Guerbet alcohol according to any one of claims 1 to 3, wherein the catalyst (A) is a catalyst including a carrier having the first component, the second component, and the third component supported thereon.

5.  The method for producing a Guerbet alcohol according to claim 4, wherein the carrier of the catalyst (A) is at least one kind selected from the group consisting of aluminum oxide, activated carbon, titanium oxide, zirconium oxide, zeolite, cerium oxide, and hydrotalcite.

6.  The method for producing a Guerbet alcohol according to any one of claims 1 to 5, wherein the raw material alcohol is a primary aliphatic alcohol having 8 or more and 18 or less carbon atoms.

7.  The method for producing a Guerbet alcohol according to any one of claims 1 to 6, wherein a base catalyst (B) is used with the catalyst (A).

8.  The method for producing a Guerbet alcohol according to claim 7, wherein an amount of the base catalyst (B) based on the total amount of the raw material alcohol is 0.1% by mol or more and 7% by mol or less.

9.  The method for producing a Guerbet alcohol according to any one of claims 1 to 8, wherein an amount of the catalyst (A) based on the total amount of the raw material alcohol is 0.01% by mass or more and 10% by mass or less for suspended bed reaction.

10. The method for producing a Guerbet alcohol according to any one of claims 1 to 9, wherein the catalyst (A) has a mass ratio of the second component to the first component of 0.10 or more and 9 or less.

11. The method for producing a Guerbet alcohol according to any one of claims 1 to 10, wherein the catalyst (A) has a mass ratio of the third component to the first component of 0.001 or more and 0.60 or less.

12. The method for producing a Guerbet alcohol according to any one of claims 1 to 11, wherein the catalyst (A) has a content of the first component of 3% by mass or more and 39% by mass or less.

13. The method for producing a Guerbet alcohol according to any one of claims 1 to 12, wherein the catalyst (A) has a content of the second component of 1% by mass or more and 20% by mass or less.

14. The method for producing a Guerbet alcohol according to any one of claims 1 to 13, wherein the catalyst (A) has a content of the third component of 0.1% by mass or more and 10% by mass or less.

15. A catalyst used for a method for producing a Guerbet alcohol, comprising a first component, a second component, and a third component below:

first component: copper,
second component: one kind selected from the group consisting of cobalt, nickel, molybdenum, and rhenium, and
third component: at least one kind selected from the group consisting of elements that are elements belonging to Groups 3 to 10 and 12 of the fourth period of the periodic table and elements belonging to Groups 3 to 7, 11, and 12 of the fifth and sixth periods of the periodic table, and are different from the element selected as the second component.

| INTERNATIONAL SEARCH REPORT | International application No. |
|---|---|
| | PCT/JP2020/047424 |

**A. CLASSIFICATION OF SUBJECT MATTER**
C07C 29/34(2006.01)i; B01J 23/75(2006.01)i; B01J 23/755(2006.01)i; B01J 23/80(2006.01)i; B01J 23/83(2006.01)i; B01J 23/847(2006.01)i; B01J 23/883(2006.01)i; B01J 23/888(2006.01)i; B01J 23/889(2006.01)i; B01J 23/89(2006.01)i; C07C 31/02(2006.01)i; C07C 31/125(2006.01)i; C07B 61/00(2006.01)n
FI:      C07C29/34; B01J23/75 Z; B01J23/755 Z; B01J23/80 Z; B01J23/83 Z; B01J23/847 Z; B01J23/883 Z; B01J23/888 Z; B01J23/889 Z; B01J23/89 Z; C07C31/02; C07C31/125; C07B61/00 300

According to International Patent Classification (IPC) or to both national classification and IPC

**B. FIELDS SEARCHED**

Minimum documentation searched (classification system followed by classification symbols)
C07C29/00-31/44; B01J23/00-23/96; C07B61/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | CN 103272608 A (CHINA RESEARCH INSTITUTE OF DAILY CHEMICAL INDUSTRY) 04 September 2013 (2013-09-04) claims, examples | 1-15 |
| A | JP 2-286638 A (KAO CORP.) 26 November 1990 (1990-11-26) claims, examples | 1-15 |
| A | WO 2018/016270 A1 (KAO CORP.) 25 January 2018 (2018-01-25) claims, examples | 1-15 |

☐ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 February 2021 (26.02.2021) | 09 March 2021 (09.03.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2020/047424

| Patent Documents referred in the Report | Publication Date | Patent Family | Publication Date |
|---|---|---|---|
| CN 103272608 A | 04 Sep. 2013 | (Family: none) | |
| JP 2-286638 A | 26 Nov. 1990 | (Family: none) | |
| WO 2018/016270 A1 | 25 Jan. 2018 | US 2019/0284120 A1 claims, examples BR 112019000839 A CN 109476569 A EP 3489212 A1 JP 2018-21011 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2286638 A